(19) Europäisches Patentamt
European Patent Office
Office européen des brevets

(11) EP 2 664 920 B1

(12) **EUROPEAN PATENT SPECIFICATION**

(45) Date of publication and mention
of the grant of the patent:
**18.09.2019 Bulletin 2019/38**

(51) Int Cl.:
***G01N 33/50*** *(2006.01)*

(21) Application number: **13168263.5**

(22) Date of filing: **17.05.2013**

(54) **Dilution based inhibition assay**

Verdünnungsbasierter Inhibitionsassay

Dosage d'inhibition à base de dilution

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.05.2012 HU 1200299**

(43) Date of publication of application:
**20.11.2013 Bulletin 2013/47**

(73) Proprietor: **Debreceni Egyetem
4032 Debrecen (HU)**

(72) Inventors:
• **Tóth, Attila, Dr.
4032 Debrecen (HU)**
• **Fagyas, Miklós, Dr.
4032 Debrecen (HU)**
• **Papp, Zoltán, Prof. Dr.
4032 Debrecen (HU)**

• **Édes, István, Prof. Dr.
4032 Debrecen (HU)**

(74) Representative: **Svingor, Adam
Danubia
Patent & Law Office LLC
POB 198
1368 Budapest (HU)**

(56) References cited:
**WO-A1-01/85985          WO-A1-2007/059966
WO-A2-02/059343     US-A- 5 260 872**

• **GRONHAGEN-RISKA C ET AL: "Competitive
inhibitor binding assay (CIBA) of captopril and
other ACE inhibitors", CLINICA CHIMICA ACTA,
ELSEVIER BV, AMSTERDAM, NL, vol. 162, no. 1,
15 January 1987 (1987-01-15), pages 53-60,
XP023396413, ISSN: 0009-8981, DOI:
10.1016/0009-8981(87)90232-4 [retrieved on
1987-01-15]**

**Description**

**FIELD OF THE INVENTION**

The description provides for a process to assess the level of reversible inhibition of an enzyme by an inhibitor, in particular in the field of assessment the effectiveness of a medical treatment. The effectiveness of an antihypertensive reversible enzyme inhibitor drug was tested. In particular the method was tested in angiotensin converting enzyme (ACE) inhibitor (ACEi) therapy. The description also relates to uses of enzyme substrates and kits for the assessment of inhibition level as well as an apparatus designed for use in a process of the present description.

**BACKGROUND ART**

The problem to assess efficiency or efficacy of enzyme inhibitor therapies obviously has been raised in the art. In these methods first of all a specific condition of the experiment is set, wherein typically enzyme activity of the patient treated is compared to the "normal" activity level of a healthy control group or a control group wherein the therapy is effective. However, this traditional method raises several issues. For example, a control group of subjects may be required, whereas said subjects necessarily have different pathophysiological properties (i.e. do not suffer in the same disease) and may show individual variability.

Another problem which often arises is related with patient compliance. Specifically, in order to monitor a treatment several samples in time should be taken. However, many patients are not ready to regularly visit a doctor which makes this assessment very unreliable.

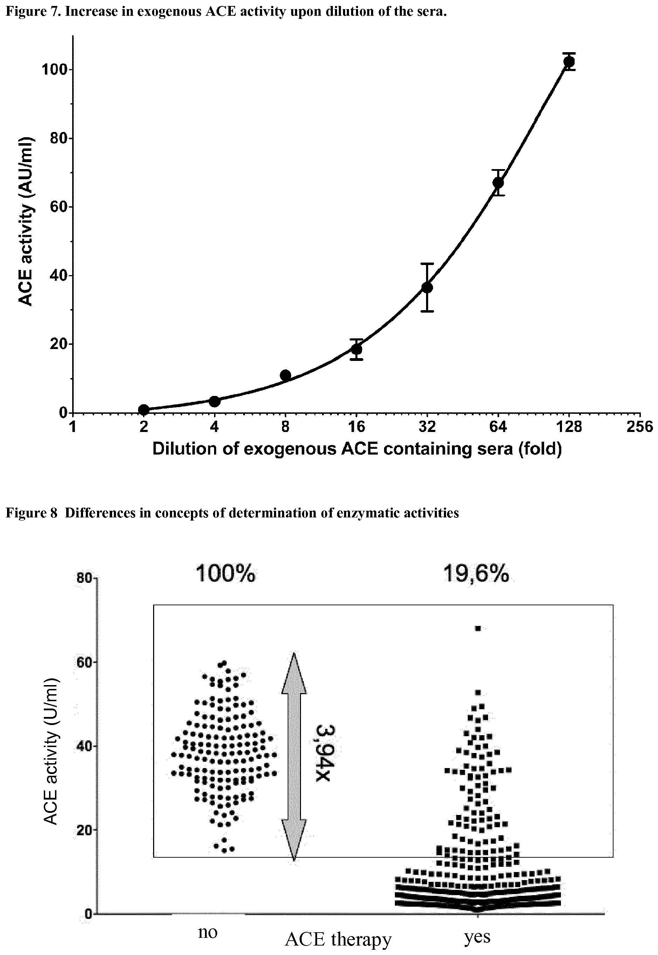

Moreover, direct activity measurement is subject to individual properties including genetic variation which, however, does not indicate the effectiveness of an inhibitor therapy and even may be unrelated. (Figure 8).

Another method to monitor whether a treatment with an enzyme is successful comes from the determination of the level of the inhibitor in the body fluid obtained or given. However, while this experiment reports on the blood level of the drug, omits to observe its actual action in the body. Further problems may arise from preliminary purification, its availability to the enzyme and that exact analytical methods are expensive in terms of time and costs.

Similar problems may also arise in every case when efficiency of the inhibition of an enzyme is to be assessed. Several attempts have been made in the art to overcome these and similar difficulties.

In US20040081957A1 the quantity of a HIV protease inhibitor is measured in its complex with a conjugate. The assay is based on competition of the conjugated inhibitor and the inhibitor to be measured.

Fyhrquist F et al. have developed an inhibitor binding assay for the measurement of angiotensin-converting enzyme level using an enalapril analog, Compound 351A which retains the stability of the parent substance after being labeled with $^{125}$I. The ACE level is assessed by reading the count of radioactivity after inhibitor binding and dilutions are used only to take a standard reference curve [Fyhrquist F. et al. "Inhibitor Binding Assay for Angiotensin-Converting Enzyme" Clin Chemistry, 1984 30(5) 696-700].

Grönhagen-Riska et al. used this inhibitor binding assay, based on specific binding of ACE to a radiolabelled inhibitor, to develop competitive inhibitor binding assays for ACE inhibitors [Grönhagen-Riska C. et al. "Competitive inhibitor binding assay (CIBA) of ACE inhibitors", Acta. Med. Scand. Suppl., 1986, 714, 43-47.]. In this assay the serum level of an ACE inhibitor is measured by adding ACE to the sample and dilution is used to minimize the effect of endogenous ACE activity. In this assay not dilution but an other inhibitor is used to measure inhibitor binding and all test samples are diluted to the same extent (same fold). Moreover, these assays are not kinetic (enzymatic activity) assays and are not suitable to measure ACE enzymatic activity.

In WO2001085985A1 the activity of enzymes with overlapping substrate specificities are measured by using samples of variable dilutions. While effectiveness of a treatment can be assessed several samples in several time-points shall be taken from the patients.

While in US6090570A dilution by a reagent is applied, a potentially defective factor is supplemented and an effective variant is added which is bound as a co-factor to the enzyme so as to mask the genetic mistake.

In WO2003025561A1 the blood-plasma concentration ratio and the plasma protein-binding ratio is measured simultaneously in differently diluted blood samples. The method, however, is rather complex and it appears that no specific enzyme inhibitor pair is examined.

In PCT/US2001/045643 a method to determine the level of inhibition of the angiotensin converting enzyme is described and the association of the level of inhibition with the appearance of angioedema. The method is different from a simple dilution based assay.

In EP02728295 it is described that the presence of inhibitors can be shown by a myeloperoxidase activity. This application, however, is unrelated to the present invention.

A method to characterize an enzyme inhibitor relationship and characterize it by determination of the inhibition constant is described e.g. in "Enzyme and transporter based drug-drug interactions" edited by K. Sandy Pang, A. David Rodrigues, Raimund M. Peter, Published by Springer, New York, 2010, ISBN: 978-1-4419-0839-1. see page 188 and references cited therein. In this methodology, however, inhibitor constants have been given for cytochrome P 450 and binding of the inhibitors is characterized. The method has not been used for the estimation of the level of the inhibitor in the sample or the effectiveness in the inhibition.

Lieberman et al. used a spectrophotometric method for assaying serum ACE and found that a serum dilution with saline solution to 1:8 was advisable for an accurate determination of enzyme activity to eliminate the nonlinear aspect of the assay and the effect of an endogenous inhibitor. This level of dilution increased the calculated serum ACE activity by a factor of 1.5 to 3. The authors note that dilutions greater than 1:8 magnify greatly any error occurring during the assay and are deemed unnecessary [Lieberman J. and Sastre A. Chest 1986;90:869-75.].

In traditional dilution based methods for determination of the inhibition of an enzyme only the inhibitor concentration is changed, whereas in the present method both the inhibitor and the enzyme are diluted.

Thus, there is still a need to use a fast and reliable method to assess the level of reversible inhibition of an enzyme by an inhibitor in a "single sample" method to assess the effectiveness of enzyme inhibitor therapies in particular to increase effectiveness of therapies based on enzyme inhibitor medicaments.

The present inventors have found that effectiveness of an enzyme inhibitor therapy can be assessed with a high reliability if sufficiently different dilution factors are applied in an enzyme activity assay on aliquots from a sample taken from a subject under therapy.

## BRIEF DESCRIPTION OF THE INVENTION

The invention relates to an *in vitro* process to assess the effectiveness of enzyme inhibition in angiotensin converting enzyme (ACE) inhibitor therapy in a subject comprising:

(a) providing a blood sample or a blood derived sample from a subject treated with an ACE inhibitor;
(b) taking at least two aliquots from the sample;
(c) diluting said aliquots by different dilution factors,
(d) measuring the ACE activity in the dilution samples;
(e) defining a high dilution factor where the effect of the ACE inhibitor becomes negligible, wherein said dilution factor is at least 50, preferably at least 100;
(f) defining a low reference dilution factor, said dilution factor being at most 10;
(g) multiplying the measured activity values in each of the diluted samples by the respective dilution factor to obtain calculated ACE activity values for each dilution samples,
(h) obtaining the level of ACE inhibition in the original sample by comparing the enzyme activity values determined at said low and high dilution factors in said dilution samples,
(i) assessing the effectiveness of medical treatment by the ACE inhibitor in said subject by evaluating the calculated level of ACE inhibition, wherein a low enzyme inhibition is an indication of an ineffective therapy, and wherein a high enzyme inhibition is an indication of an effective therapy.

Preferably, the high dilution factor, wherein the effect of the ACE inhibitor is negligible, is at least 100 or at least 200, preferably at least 300 or at least 350 or highly preferably at least 400,
and wherein the reference low dilution factor is at most 20 or at most 10 or highly preferably at most 5 or at most 4, and/or the ratio of dilution factors is thus at least 10 or preferably at least 30 or at least 70 or at least 100.

In a preferred embodiment,
in step (i) enzyme inhibition is considered as insufficient in a subject having a level of inhibition, given in percentage, lower than 90% and effective in a subject having a level of inhibition of at least 90%,
thereby assessing the effectiveness of said ACE inhibitor therapy in said subject.

## DEFINITIONS

As used herein the singular forms "a", "an" and if context allows "the" include plural forms as well unless the context dictates otherwise.

"Isolated" means altered "by the hand of man" from the natural state. If a molecule or composition occurs in nature, it has been "isolated" if it has been changed and/or removed from its original environment.

The term "comprises" or "comprising" or "including" are to be construed here as having a non-exhaustive meaning and allow the addition or involvement of further features or method steps or components to anything which comprises the listed features or method steps or components.

The expression "consisting essentially of' or "comprising substantially" is to be understood as consisting of mandatory features or method steps or components listed in a list e.g. in a claim whereas allowing to contain additionally other features or method steps or components which do not materially affect the essential characteristics of the use, method, composition or other subject matter. It is to be understood that "comprises" or "comprising" or "including" can be replaced herein by "consisting essentially of' or "comprising substantially" if so required without addition of new matter.

The term "single source" or samples from a "single source" is to be understood herein that there exists or there existed a composition of matter from which said samples are or were taken form or said samples have been prepared or derived from samples taken from said composition of matter, preferably at essentially at the same time. Preferably the samples from a single source have essentially identical composition.

"Essentially the same time" is to be understood herein as sufficiently close in time so that the composition of the single source was not essentially changed i.e. it was still essentially identical.

"Essentially identical composition" of two samples, e.g. two samples taken from a composition of matter in different time points, means that the composition thereof is sufficiently identical to perform reliably comparable measurements in or from the two samples.

**BRIEF DESCRIPTION OF THE FIGURES**

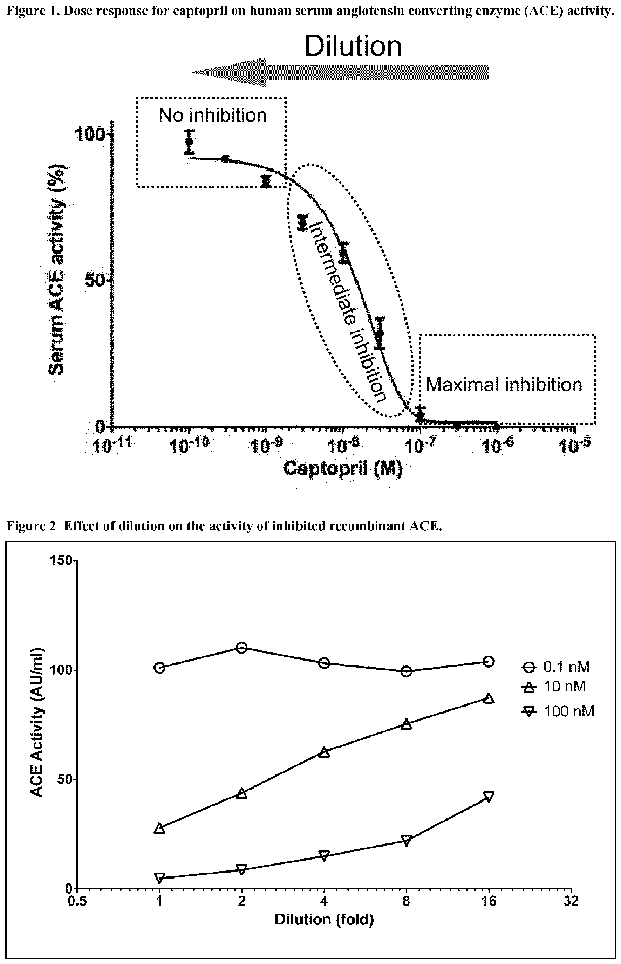

**Figure 1. Concentration response for captopril on human serum angiotensin converting enzyme (ACE) activity.**

Increasing concentrations of captopril antagonized human serum ACE activity in a nonlinear fashion. The clinical goal of the use of enzyme inhibitors (such as the ACE inhibitor captopril) is to achieve high level of enzyme inhibition. However, the dose of the enzyme inhibitor drugs is limited by the appearance of side effects. Inhibitor concentration (such as ACE in this particular case) is therefore most probably is in the submaximal level (labelled by intermediate level on the figure) in the clinical practice. This range is characterized by the fact that small changes in the inhibitor concentration can evoke large differences in the actual inhibitor effects. Taking the example shown on the figure one may calculate that the level ACE inhibition decreases from 68% to 30% when captopril concentration decreases from 30 nM to 3 nM (10-fold dilution), respectively.

**Figure 2. Effect of dilution on the activity of inhibited recombinant ACE.**

Recombinant ACE was mixed with the ACE inhibitor captopril (0.1 nM, 10 nM and 100 nM, concentrations are indicated), then ACE activity was measured in the undiluted (1-fold) and in the diluted (up to 16-fold) solutions. There were little effects of dilution when ACE activity was inhibited by a low effectiveness (e.g. at 0.1 nM). However, a robust increase in ACE activity upon dilution was observed when captopril was present in a clinically more relevant concentration (10 nM). Finally, the robust increase in ACE activity was only observed at higher dilution factors (e.g. 16-fold) in the case of a high dose of captopril (100 nM).

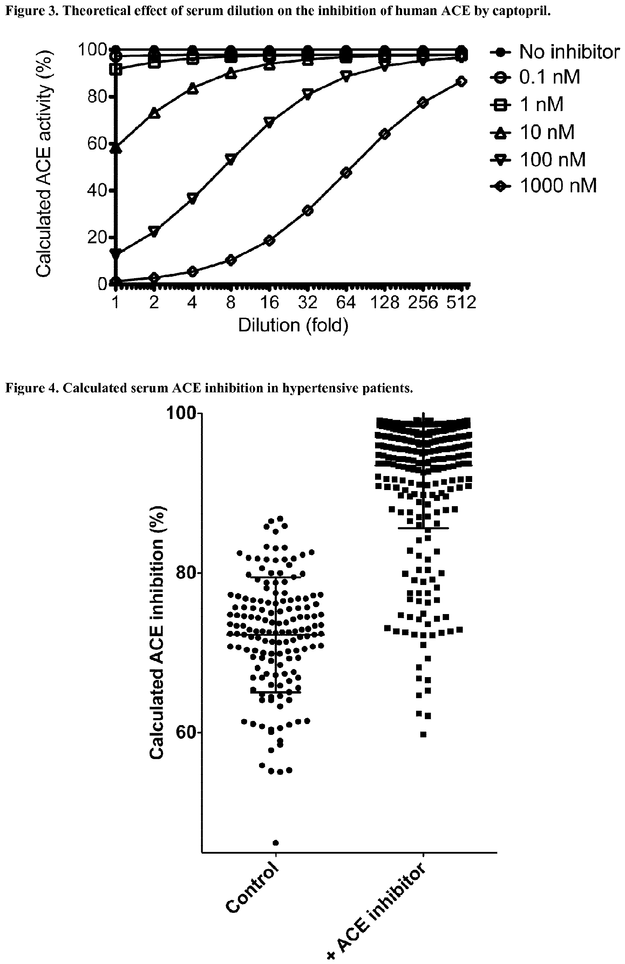

**Figure 3. Theoretical effect of serum dilution on the inhibition of human ACE by captopril.**

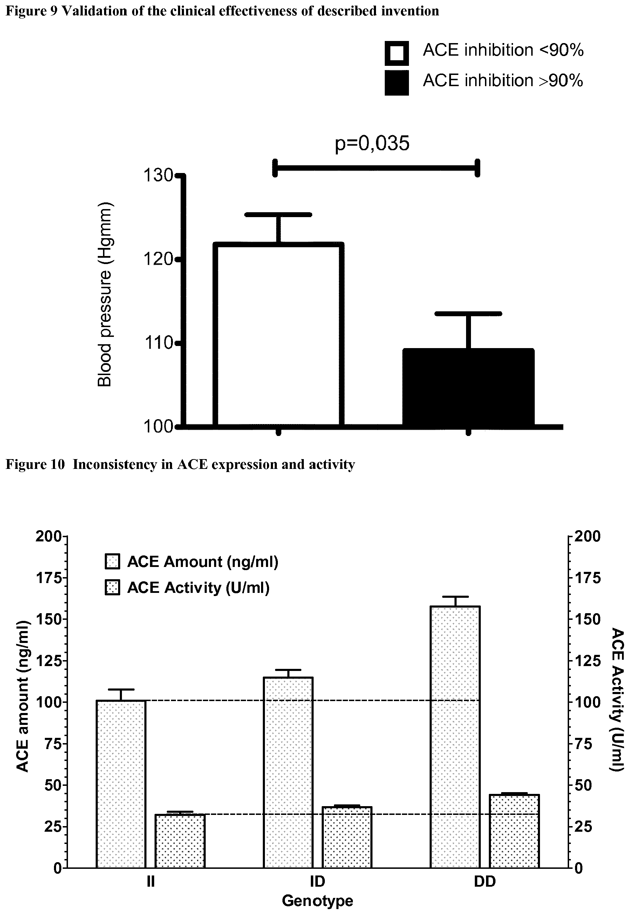

Equation 1 describes a general model of reversible enzyme inhibition, in which the actual enzyme activities are depending on the dilution factor used to generate reaction samples. Theoretical enzyme activity values were calculated at supposed dilution values in the range of 1 - 512. Initial captopril concentration was defined in 0 - 1000 nM range (represented by the different symbols). ACE serum concentration was estimated based on measured data presented in Figure 10. The data was fitted according to equation 1. The figure suggests that the increase in enzyme activity depends on the dilution factor, which can be defined based on the predetermined (known) properties (e.g. Ki and cooperativity) of the enzyme-inhibitor interaction. In particular, inhibition level can be estimated by comparing enzyme activity values at low (enzyme is in its inhibitor bound form) and high dilution factors (enzyme is dominantly in its free, active form). Level of inhibition can be estimated when enzyme activity determined at low dilution factor is compared to the enzyme activity at high dilution factors.

**Figure 4. Calculated serum ACE inhibition in hypertensive patients.**

The applicability of the processes claimed in this application was proved using the angiotensin converting enzyme and patients treated with its inhibitors. Activity of serum ACE was tested in human blood samples. Hypertensive patients were involved. Some of the patients were taking ACE inhibitors (labelled by + ACE inhibitor), while other patients did not have ACE inhibitor prescribed (labelled as Control). Symbols are representing the calculated serum ACE inhibition is shown in individual patients without (control) and with ACE inhibitor therapy. Symbols represent the mean of the determinations and bars show the standard deviation. It is apparent that there are some patients from the treated group with similar ACE activities than that of in treated patients, but it is also apparent that the majority of patients with ACE inhibitor prescribed had lower enzyme activities.

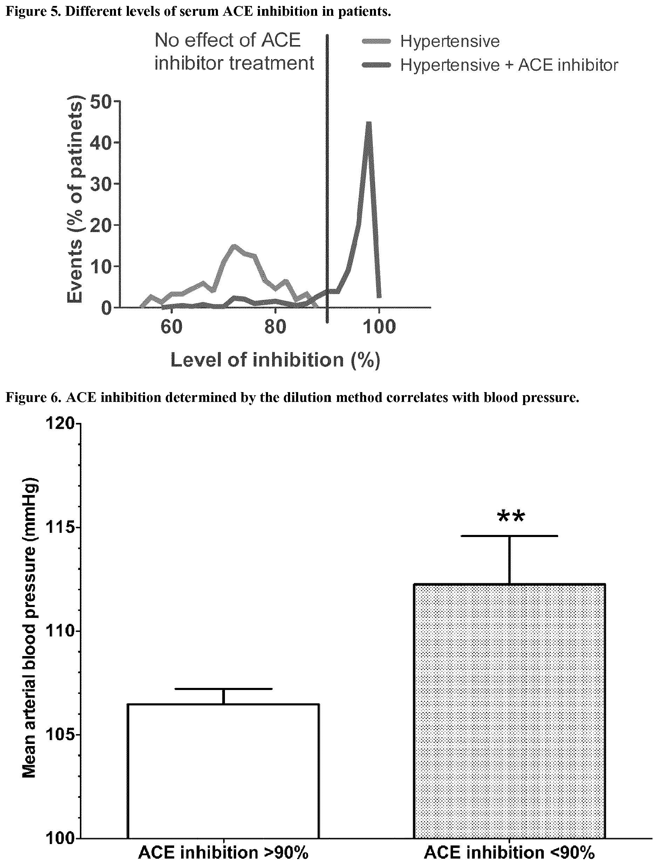

**Figure 5. Different levels of serum ACE inhibition in patients.**

When the level of ACE inhibition was assessed by the method described in this application a surprising finding emerged. Untreated patients showed a significant (about 75%) inhibition without taking ACE inhibitors. This suggested the presence of an endogenous reversible inhibitor. On the other hand, patients with ACE inhibitor therapy tended to have a much higher level of ACE inhibition. Data have been plotted as frequency distribution (histogram). The line represents the chosen arbitrary value (90%) hypothetically representing the threshold for effective ACE inhibitor therapy.

**Figure 6. ACE inhibition determined by the dilution method correlates with the respective clinical target of the treatment (blood pressure).**

ACE inhibition was assessed by performing the process described in this application. ACE activity was determined at 4 and 400-fold dilutions of serum samples, besides to the measurement of blood pressure. Apparent ACE inhibition was calculated by the ratio of the values upon 400-fold and 4-fold dilutions. Mean arterial blood pressure of the patients with apparent ACE inhibition below (n=68) or above (n=318) 90% are shown. Bars represent the mean and standard error of the mean, while asterisks represent the significant difference between the two groups (p=0.003). It was found that patients with apparently insufficient ACE inhibition had higher blood pressure values, suggesting that the invention can effectively be used to estimate clinical effectiveness of the treatment targeting an enzyme with reversible inhibitors.

**Figure 7. Increase in exogenous ACE activity upon dilution of the sera.**

The process described in this application suggests that desired dilution factors can be predetermined taking into account of the predetermined Ki of an inhibitor and the concentration-response of the inhibitor on the activity of an enzyme. Exogenous ACE activity was measured to determine ACE inhibition in the sera of a patient on ACE inhibitor therapy. Data represent the results of 3 determinations (mean±SD). The results suggest that activity of inhibited ACE can significantly increase upon dilution. In particular ACE activity was about 1 AU/ml without dilution and increased to more than 100 AU/mg upon high dilution (128-fold).

**Figure 8. Differences in concepts of determination of enzymatic activities**

The effectiveness of an enzyme inhibitor treatment is traditionally characterized by comparing the enzyme activities of a treated group (where inhibition is expected) to a control population, where inhibition is not expected. Then a reference range is established for the enzyme based on its activity in the control population and enzyme activity values are related to this reference range. However, untreated individuals tend to be different from patients with a certain disease and the activity of an enzyme may change upon the progression of the disease. Here we compared two groups of hypertensive patients according to their medication, control group was defined as patients without prescribed ACE inhibitor drug (153 patients) while the treated group contained individuals with ACE inhibitor prescribed (387 patient). It was apparent that a significant variance (about 4-fold range) was found in the ACE activity of the untreated, control group, In addition there was an about 20% overlap between these groups, which makes it very uncertain whether the patient had a relatively high basal ACE activity which was successfully inhibited, or the patient had insufficient ACE inhibition.

**Figure 9. Validation of the clinical effectiveness of described invention**

Those patients were contacted where ACE inhibition was lower than 90%, determined from single blood samples according to this present invention. Patients were asked to pay a special attention to their medication, in particular to administer the prescribed ACE inhibitor properly and to come back for a second visit. In some cases patients had enzyme inhibition higher than 90% at this second visit. A strong relation was found when their determined blood pressure level was related to the defined biochemical effectiveness of the medication. In particular, blood pressure was lower when ACE inhibition was higher than 90%, compared to the blood pressure of the same patients when ACE inhibition was lower than 90%. Averaged values are shown (mean±SEM).

**Figure 10. Inconsistency in ACE expression and activity**

Serum ACE expression was determined by an ELISA assay, while serum ACE activity was determined by FAPGG hydrolysis in the same samples. In parallel, patient's genotype was also determined. Patients with DD genotype had an about 58% higher ACE expression in the sera compared to the patients with II genotype, while their serum ACE activity was significantly less affected by the genotype.

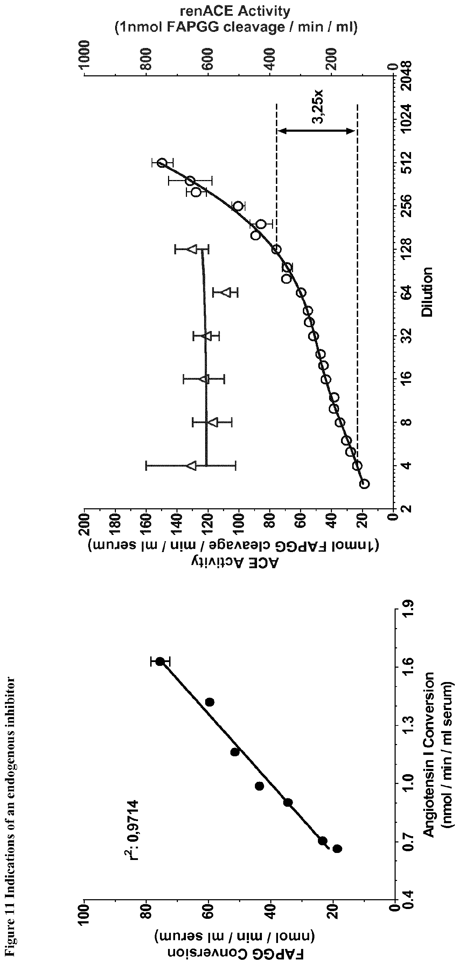

**Figure 11. Indications of an endogenous inhibitor**

The inconsistency between ACE expression and activity may be related to an inappropriate method to detect slight changes in ACE activity. ACE activity was measured by the artificial substrate (FAPGG) and by the endogenous substrate angiotensin 1. A strong correlation between activities determined by these substrates was found, suggesting that the measurement of activity based on FAPGG hydrolysis was appropriate. Next, the effect of dilution on the enzyme activity was tested. It was found that calculated serum ACE activity is increased in parallel with the dilution in a non-linear fashion, in contrast with the ACE activity when recombinant enzyme was tested without components present in the serum. This observation suggested that an endogenous inhibitor is present in the serum, in accordance with the claims of this application.

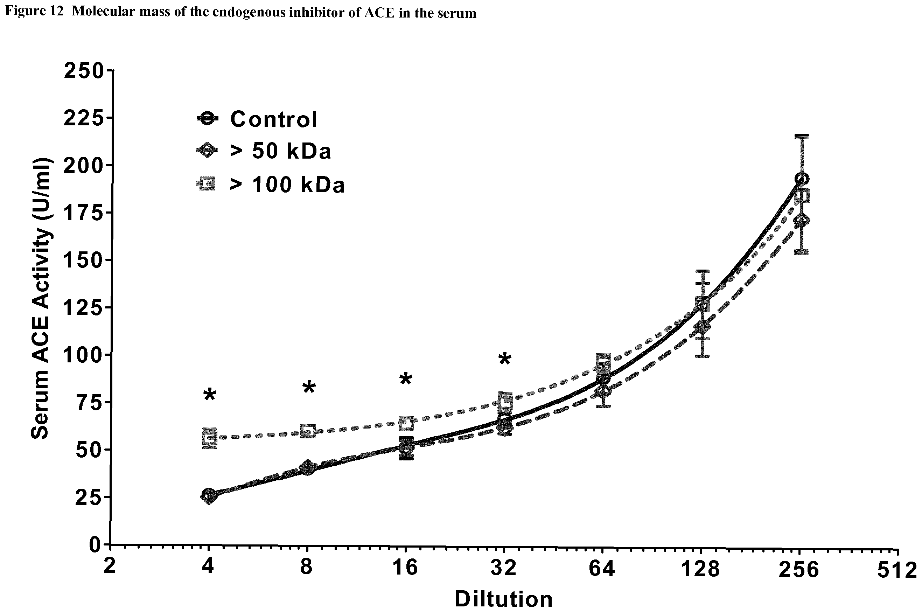

**Figure 12. Molecular mass of the endogenous inhibitor of ACE in the serum**

Serum samples of healthy, untreated individuals were filtrated through filters which were impermeable for proteins higher than 50 kDa or higher than 100 kDa. There was no effect of filtration through the 50 kDa filter, but filtration through the 100 kDa filter resulted in a significant change in the enzyme activities upon increasing dilutions. In particular, there was a significantly higher activity in samples filtrated samples (e.g. 100 kDa filter) at low dilutions.

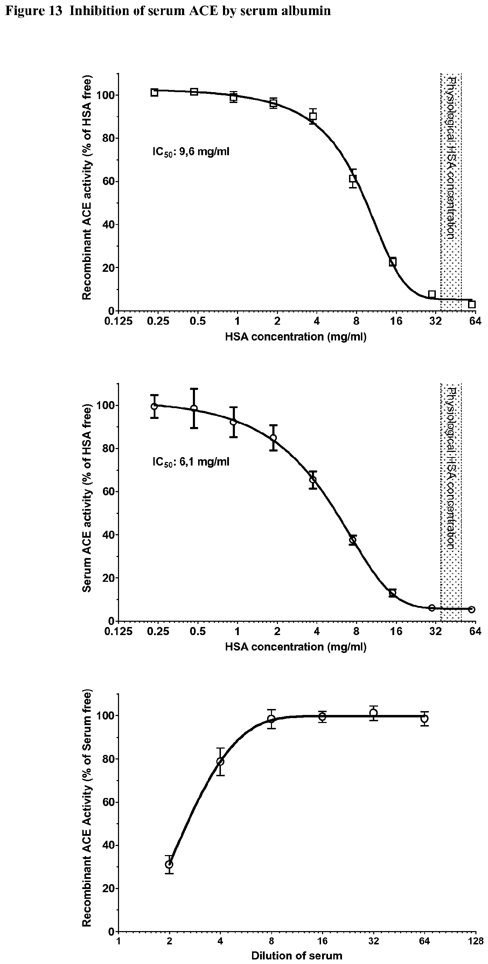

**Figure 13. Inhibition of serum ACE by serum albumin**

Efforts to identify the endogenous inhibitor with a molecular mass of less than 100 kDa, and higher than 50 kDa resulted evidences for a direct interaction between the serum ACE and the serum albumin. Effect of serum albumin on recombinant ACE was tested first. Increasing concentrations of human serum albumin inhibited the recombinant ACE activity in a dose-dependent manner, with a Ki value of 9.6 mg/ml. Taking into account that albumin content of the human sera is in the range of 40-60 mg/ml, physiological inhibition of serum ACE may be explained by the presence of the serum albumin. To test this hypothesis, ACE was purified from human sera and the effects of human serum albumin were tested. Human serum albumin inhibited purified human serum ACE in a similar fashion than that of recombinant ACE. Finally, the current description claims that the presence of an inhibitor can be detected by adding exogenous enzyme to the reaction mixture. This claim was tested by making serial dilutions of a sample containing the recombinant ACE and human serum albumin. A robust increase in calculated ACE activities was found even at low dilution ranges (e.g. at 2-fold and at 8-fold dilution).

## DETAILED DESCRIPTION OF THE INVENTION

The present inventors have created a fast and simple method to assess the effectiveness of the inhibition of an enzyme in an appropriate sample. According the present method it is not necessary to take a series of samples or taking samples multiple times and determination is made from a single sample taken preferably from a patient on a single occasion. This allows for example the assessment of the effectiveness of enzyme inhibitor therapies even without monitoring the inhibitor level in the patient by taking samples at multiple times.

The concentration-response relationship between inhibitors and enzymes are characterized by a sigmoidal concentration-response curve, such as shown on Fig. 1. representing our measurements. The figure shows the important phases of enzyme inhibition, relevant to this invention: the inhibition is maximal under saturating conditions (each enzyme molecule is bound to an inhibitor); an intermediate level of inhibition can be observed in the case of lower inhibitor concentrations; while the inhibitor become ineffective in case of even lower concentrations (unaltered, maximal enzyme activity). The clinical effects of medical drugs are usually intermediate, e.g. inhibition of the target is significant, but the level of antagonism does not reach complete inhibition.

The theoretical basis of the observation is that the stability of any reversible inhibitor-enzyme complex can be characterized by a constant (Ki), while the actual concentrations of the free enzyme (E), free inhibitor (I) and the enzyme-inhibitor complex (EI) can be calculated as follows (equation 1):

$$\mathbf{Ki = (E*I)/EI} \qquad (1)$$

The ratio of the concentrations of the inhibited enzyme (EI) to the total concentration of the enzyme (E+EI), representing the actual level of inhibition (Inh) can also be calculated (equation 2):

$$\mathbf{Inh = EI/(E+EI)} \qquad (2)$$

The basis of the present invention is that it appears to be theoretically possible to estimate the maximal enzyme activity of a sample by applying a sufficiently high dilution. Upon these conditions (high dilution) the concentrations of the enzyme-inhibitor (EI) complexes may be negligible compared to the concentration of the free enzyme (EI<<E). Taking the example of a reversible inhibitor: if enzyme activities are measured in at least two dilutions of the same sample then the most concentrated sample gives us the inhibited enzyme activity (Einh), while enzyme activity determined upon higher dilution of the same sample may represent the enzyme activity in conditions when the inhibitor dissociates from the enzyme, therefore unmasking its activity, which is similar to those in the absence of the inhibitor (such as when the same patient would not have taken his medication). As a consequence, the maximal enzyme activity of the sample (Emax, when E>>EI) can be estimated based on the values determined under diluted conditions (under these conditions the majority of the enzyme is present in its active (dissociated) form), while the actual level of enzyme inhibition (when significant concentration of EI is present) can be estimated in the least diluted sample (under these conditions a significant proportion of the enzyme is present in its inhibited (enzyme-inhibitor complex) form).

The actual level of reversible inhibition (Inh) within a single sample may therefore be estimated according to equation 3:

$$\mathbf{Inh = Einh/Emax} \qquad (3)$$

The low and high dilution factors can be determined e.g. as follows.

The high dilution factor is preferably set to a value where the effect of the reversible inhibitor becomes negligible.

To estimate the necessary dilution factor the effects of a selected inhibitor is tested. In particular, the concentration-response curve for said inhibitor may be taken. By fitting the data with a nonlinear fit (curve) based on equation 1 the stability constant for the enzyme can be obtained.

Further factors are the appropriate concentrations of the enzyme and the inhibitor. The inhibitor concentration is affected by the amount of enzyme inhibitor drug in the sample as well as the metabolism of the drug, drug-drug-interactions. However, inhibitor concentrations are still usually limited in terms of the maximal concentration, because of limited availability of the pills containing the drug and potential side effects. Thus, maximal drug concentrations can be estimated. The enzyme concentration can be obtained experimentally.

Having determined some of the values mentioned in the equations above, it is possible to construct models for the effects of dilution. For example, equation 1 can be used to calculate the effects of dilutions on enzyme activity. Values for calculated enzyme activity [% of active enzyme (E) related to the total enzyme concentration (E+EI)] can be obtained as the function of the dilution factor (affecting the concentrations of the components of the solution (E, EI, I) at different initial inhibitor concentrations, covering the full physiological range (0.1 - 1000 nM) (Fig. 3).

Thereby the appropriate dilution factors can be determined.

Thus, preferably the high dilution factor in case of a sample in which the inhibitor bound form is negligible is at least 20 or at least 30 or at least 50 or at least 100 or at least 200, preferably at least 300 or at least 350 or highly preferably at least 400.

Thus, preferably the low dilution factor in case of a sample in which the active form is considered as negligible is at most 20 or at most 10 or highly preferably at most 5 or at most 4.

The ratio of dilution factors is thus at least 10 or preferably at least 30 or at least 70 or at least 100.

In terms of the invention it is critical to use as much dilution as possible to estimate uninhibited (maximal) enzyme activities. According to the experience of the Inventors the inhibition can be very accurately estimated by values at 1 and 512-fold dilutions (Table 1).

The present process is an alternative way to determine the level of reversible inhibition in experimental samples. The major advantage of this process is that it may be used to directly assess the level of reversible inhibition in a single sample, without the need to compare the values to any other samples. Directly assessing the level of reversible inhibition may be desired when a suitable control is not available or exact inhibition values are required from a single sample. These applications may include testing of the effectiveness of a medication aiming at inhibiting an enzyme by the administration of a reversible inhibitor of said enzyme at the biochemical level. The present inventors have illustrated the method of the process according to the description on the renin-angiotensin-aldosterone system, in a clinical setup, where effectiveness of the angiotensin converting enzyme (ACE) inhibitor therapy has been evaluated.

Angiotensin-converting enzyme (ACE) is a zinc-metalloendodipeptidase that catalyses angiotensin I to angiotensin II conversion, and takes part in the metabolism of other peptides like bradykinin [Corvol P, Michaud A, Soubrier F, Williams TA. Recent advances in knowledge of the structure and function of the angiotensin I converting enzyme. J Hypertens Suppl 1995;13:S3-10.]. ACE has two isoenzymes: a somatic and a testicular form [Hubert C et al. J Biol Chem 1991;266:15377-83.]. The somatic form of ACE is released into the circulation by the ACE secretase [Oppong SY et al. Biochem J 1993;292 (Pt 2):597-603., Wei L et al. J Biol Chem 1991;266:5540-6.]. ACE is a member of the renin-angiotensin-aldosterone system (RAAS), which is an important regulator of blood pressure and salt-water homeostasis [Pfeffer MA et al. N Engl J Med 1992; 327:669-77].

ACE inhibitors represent one of the most effective and commonly used therapeutic modulators of RAAS. ACE inhibitors significantly reduced mortality in thousands of patients in various cardiovascular pathologies. In particular ACE inhibitors, according to several megatrials, reduce the risk of cardiovascular death, nonfatal myocardial infarction or cardiac arrest in stable coronary heart disease [Fox KM. Lancet 2003;362:782-8.], they improve prognosis [GISSI-3 trial. J Am Coll Cardiol 1996;27:337-44.] and reduce the 5-week mortality [ISIS-4 trial, Lancet 1995;345:669-85.] after myocardial infarction. These drugs reduce heart failure mortality [Cohn JN, et al. N Engl J Med 1991;325:303-10.] and inhibit left ventricular remodeling [Greenberg B et al. Circulation 1995;91:2573-81.]. ACE inhibitors delay the manifestation of hypertension [Luders S, et al. J Hypertens 2008;26:1487-96.] and reduce left ventricular mass index in left ventricular hypertrophy [Cuspidi C et al. J Hypertens 2002;20:2293-300.]. They are also known to reduce the incidence of microalbuminuria and the risk of diabetic nephropathy in type 2 diabetes [Ruggenenti P, N Engl J Med 2004;351:1941-51.], and further reduce the likelihood of newly diagnosed diabetes mellitus [Hoogwerf BJ, et al. Cleve Clin J Med 2000;67:287-93.]. These clinical trials strongly suggested a prominent role of RAAS including ACE in the pathomechanism of these diseases. Studies were performed to study the relationship between ACE expression on cardiovascular pathologies. It was found, that ACE expression in controlled by an insertion/deletion (I/D) polymorphism in the ACE gene [Rigat B et al. J Clin Invest 1990;86:1343-6.]. Accordingly increased amount of ACE produced in the presence of the allele D was considered as a cardiovascular risk factor [Cambien F et al. Nature 1992;359:641-4.; Staessen JA et al. J Hypertens 1997;15:1579-92.].

Hundreds of studies tested the value of ACE genotypes in risk assessment and optimization of ACE inhibitor therapy. These efforts resulted in the confirmation of the role of genotype in ACE expression (patients with DD genotype appears to have 1.5 fold [Rigat B et al. J Clin Invest 1990;86:1343-6.] higher serum ACE expression than patients with II genotype) but mostly failed to provide a positive correlation of serum ACE expression and cardiovascular diseases, even if ACE inhibition appears to be a successful treatment option.

These data suggest that differences in ACE expression are well tolerated at the individual level, further supported by the fact, that the allele D is the more frequent (50%) [Lindpaintner K. et al. N Engl J Med 1995;332:706-11.], and evolutionarily not suppressed.

The goal of our study was to identify the relationship between ACE genotype, expression a physiological activity. Performing the process claimed in this present invention we found that ACE activity is controlled by an endogenous inhibitor, which is suppressed serum ACE activity by at least 75% in human, buffering ACE activity in a wide range of expression levels.

## EXAMPLES

Endogenous angiotensin converting enzyme (ACE) activity was measured in sera drawn from a healthy volunteer upon in vitro addition of increasing doses of the ACE inhibitor captopril and the concentration-response relationship illustrated on Fig. 1. It is clearly seen that the inhibition is maximal under saturating conditions, while the inhibitor become ineffective in case of even lower concentrations (unaltered, maximal enzyme activity). The clinical effects of medical drugs are usually intermediate, e.g. inhibition of the target is significant, but the level of antagonism does not reach complete inhibition.

The feasibility of the method was tested at first when recombinant ACE was mixed with an ACE inhibitor (captopril) and then serially diluted (Fig. 2). The results have proven the feasibility of the method. Dilution of the mixtures resulted in an apparent increase in ACE activity, representing the dissociation of the inhibitor (I) and the enzyme (E), and therefore decreasing the concentration of the inhibited ACE (EI). Moreover, it was also apparent, that the increase in apparent ACE activity is related to the level of inhibition: there is no change in ACE activity when initial inhibition is negligible (captopril concentration 0.1 nM), there is a robust increase even at low dilution factors when the initial inhibitor dose is close to its Kd (e.g. results in the case of 10 nM captopril), and finally, increasing the initial concentration of the inhibitor to maximal inhibition levels (e.g. 100 nM captopril) requires high dilution factors (more than 16-fold) to unmask enzyme activity (Fig. 2).

Next, the method was tested in a real world setting, in particular in a clinical scenario.

### Estimating the inhibition of an endogenous enzyme

Determination of the dilution factor where the effect of the reversible inhibitor becomes negligible.

In the case of ACE inhibitor therapy the effect is the inhibition of ACE activity, which is supposed to be characterized by equation 1.

To estimate the necessary dilution factor the effects of a selected inhibitor was tested on the ACE activity. In particular, the concentration-response for captopril to inhibit ACE activity, is shown on Fig. 1. Fitting the data with a nonlinear fit (curve) based on equation 1 the stability constant for the human serum ACE-captopril complex (Ki) is 14.8 nM.

Further important factors are the appropriate concentrations of the enzyme (ACE) and the inhibitor (e.g. captopril). The inhibitor concentration is being affected by the amount of ACE inhibitor drug taken, the metabolism of the drug, drug-drug-interactions, etc, a full set of reasons to investigate the effectiveness. However, inhibitor concentrations are still usually limited in terms of the maximal concentration, because of limited availability of the pills containing the drug and potential side effects. It is therefore may be estimated that maximal drug concentrations are not much higher what is necessary to fully inhibit ACE. In the case of captopril it appears in the 100 nM- 1000 nM range.

ACE concentration has been estimated by direct measurement of the ACE content of human plasma drawn from 110 volunteers. Serum ACE concentration was $155 \pm 6$ ng/ml (0.86 nM).

Having defined some of the values mentioned in the equations of the description, it is possible to construct models for the effects of dilution. In this particular case equation 1 has been used to calculate the effects of dilutions on the ACE activity. Variable parameter: inhibitor concentration, constant parameters and values: ACE concentration = 0.86 nM; Ki = 14.8 nM. Values for calculated ACE activity (% of active enzyme (E) related to the total enzyme concentration (E+EI)) were plotted as the function of the dilution factor (affecting the concentrations of the components of the solution (E, EI, I) at different intial inhibitor concentrations, covering the full physiological range (0.1 - 1000 nM) (Fig. 3).

It appears, that the dilution where inhibited (inhibitor bound) form of ACE becomes negligible compared to free (dissociated, maximal activity) ACE is in the range of 128-512-fold, depending on the expected concentrations of the captopril.

Thus, preferably the high dilution factor in case of a sample in which the inhibitor bound form is negligible is at least 200, preferably at least 300 or at least 350 or highly preferably at least 400.

Thus, preferably the low dilution factor in case of a sample in which the active form is considered as negligible is at most 20 or at most 10 or highly preferably at most 5 or at most 4.

The ratio of dilution factors is thus at least 10 or preferably at least 30 or at least 70 or at least 100.

Important to note that in terms of the invention it is critical to use as much dilution as possible to estimate uninhibited (maximal) enzyme activities. Indeed, the inhibition can be very accurately estimated by values at 1 and 512-fold dilutions (Table 1). This theoretical consideration has been tested in a clinical setup where patients were treated with various inhibitors of ACE. It was confirmed that dilution factors suggested are sufficient to determine ACE inhibition.

**Table 1. Theoretical values for the inhibition of ACE activity**

|  | Estimated inhibition 1 and 512 dilution (% of uninhibited) | Estimated inhibition 4 and 400 dilution (% of uninhibited) | Theoretical inhibition (% of uninhibited) |
|---|---|---|---|
| No captopril | 0 | 0 | 0 |
| 0,1 nM; captopril | 0.7 | 0.2 | 2.7 |
| 1 nM; captopril | 6.3 | 1.6 | 8.2 |
| 10 nM; captopril | 40.2 | 14.3 | 41.5 |
| 100 nM; captopril | 86.9 | 62.2 | 87.4 |
| 1000 nM; captopril | 98.3 | 93.5 | 98.6 |

The level of inhibition is calculated according to the method described in Fig. 2. Values for inhibition are also given when activity is measured at 1, 4, 400 and 512-fold dilutions.

Setting up an assay to measure enzyme activity

ACE activity should be measured accurately even after dilutions up to 512-fold. Development of methods to determine enzyme activity may not be easy. In particular, their accuracy may be inversely related to the cost of determination. The bottom line in terms of the invention is to measure enzyme activity without significant inhibition in the presence of an inhibitor. This requires high dilution factor. Several assay methods were tested to carry out the invention. Finally, a photometric method originally described by Simonetta Ronca-Testoni (Clinical Chemistry (1986), 29(6), 1093-1096) was used and optimized to our equipment.

A detailed description of the assay is given in the Methods section (ACE activity assay).

This method was suitable to accurately determine enzyme activities in a range of 4 to 400-fold diluted sera. Although calculation based on the activities at 4-fold and 400-fold dilution tend to underestimate the inhibition, the obtained values may be used to evaluate the effectiveness of the treatment.

**Table 2. Determination of serum ACE inhibition in single blood samples**

| Patient's code | ACE activity | | | | Activity ratio(%) | Inhibition (%) |
|---|---|---|---|---|---|---|
|  | 4-fold dilution | | 400-fold dilution | | | |
|  | Measured | Calculated | Measured | Calculated | | |
| A 02 | 1.46 | 5.83 | 0.37 | 147.3 | 4.0 | 96 |
| A 03 | 2.14 | 8.57 | 0.51 | 202.41 | 4.2 | 95.8 |
| A 04 | 1.15 | 4.61 | 0.52 | 208.54 | 2.2 | 97.8 |
| A 05 | 4.64 | 18.56 | 0.37 | 149.61 | 12.4 | 87.6 |
| A 06 | 1.99 | 7.98 | 0.38 | 153.64 | 5.2 | 94.8 |
| A 08 | 2 | 8.02 | 0.47 | 189.14 | 4.2 | 95.8 |
| A 10 | 0.65 | 2.62 | 0.3 | 120.71 | 2.2 | 97.8 |

(continued)

| Patient's code | ACE activity | | | | Activity ratio(%) | Inhibition (%) |
|---|---|---|---|---|---|---|
| | 4-fold dilution | | 400-fold dilution | | | |
| | Measured | Calculated | Measured | Calculated | | |
| A 11 | 0.66 | 2.66 | 0.5 | 198.27 | 1.3 | 98.7 |
| A 14 | 1.56 | 6.22 | 0.35 | 141.28 | 4.4 | 95.6 |
| A 17 | 1.35 | 5.38 | 0.32 | 127.78 | 4.2 | 95.8 |
| A 18 | 0.45 | 1.81 | 0.42 | 167.66 | 1.1 | 98.9 |
| A 20 | 2.58 | 10.33 | 0.21 | 85.23 | 12.1 | 87.9 |
| A 21 | 1.15 | 4.59 | 0.26 | 102.05 | 4.5 | 95.5 |
| A 25 | 0.42 | 1.7 | 0.32 | 128.1 | 1.3 | 98.7 |
| A 27 | 1.22 | 4.89 | 0.44 | 174.45 | 2.8 | 97.2 |
| A 29 | 0.76 | 3.05 | 0.33 | 131.27 | 2.3 | 97.7 |
| A 30 | 1.11 | 4.44 | 0.6 | 239.22 | 1.9 | 98.1 |
| A 32 | 0.56 | 2.23 | 0.35 | 140.06 | 1.6 | 98.4 |
| A 33 | 1.63 | 6.52 | 0.41 | 162.98 | 4.0 | 96.0 |
| A 34 | 0.38 | 1.53 | 0.19 | 75.45 | 2.0 | 98.0 |
| A 35 | 8.17 | 32.67 | 0.39 | 156.49 | 20.9 | 79.8 |
| A 37 | 2.4 | 9.62 | 0.36 | 144.62 | 6.7 | 93.3 |
| A 38 | 0.65 | 2.62 | 0.32 | 129.55 | 2.0 | 98.0 |
| A 39 | 1.15 | 4.61 | 0.39 | 157.53 | 2.9 | 97.1 |
| A 44 | 0.65 | 2.61 | 0.18 | 70.97 | 3.7 | 96.7 |
| A 47 | 3.28 | 13.13 | 0.35 | 141.09 | 9.3 | 90.7 |
| A 50 | 9.45 | 37.82 | 0.37 | 146.56 | 25.8 | 74.2 |
| A 51 | 0.59 | 2.35 | 0.38 | 151.74 | 1.5 | 98.5 |
| A 52 | 0.63 | 2.51 | 0.4 | 159.04 | 1.6 | 98.4 |
| A 53 | 0.5 | 1.98 | 0.43 | 173.97 | 1.1 | 98.9 |
| A 55 | 1.16 | 4.62 | 0.41 | 163.83 | 2.8 | 97.2 |
| A 56 | 10.53 | 42.1 | 0.47 | 187.48 | 22.5 | 77.5 |
| A 61 | 4.36 | 17.45 | 0.32 | 129.46 | 13.5 | 86.5 |
| A 65 | 0.53 | 2.11 | 0.32 | 126.19 | 1.7 | 98.3 |
| A 66 | 3.68 | 14.74 | 0.36 | 144.26 | 10.2 | 89.8 |
| A 67 | 0.99 | 3.97 | 0.39 | 154.32 | 2.6 | 97.4 |
| A 69 | 3 | 11.99 | 0.38 | 152.39 | 7.9 | 92.1 |
| A 70 | 6.57 | 26.27 | 0.41 | 165.44 | 15.9 | 84.1 |
| A 71 | 1.56 | 6.23 | 0.28 | 113.87 | 5.5 | 94.5 |
| A 72 | 9.63 | 38.52 | 0.24 | 95.92 | 40.2 | 59.8 |
| A 73 | 0.25 | 1.02 | 0.13 | 50.87 | 2.0 | 98.0 |

Validation of the method in clinical samples

There were 539 patients recruited in the study. All of the patients have taken antihypertensive therapy, which included ACE inhibitors in 386 patients. The experimental data from the first 50 patients are shown on Table 2. The measured enzyme activities were multiplied by the dilution factor (4 or 400) to estimate the activities in the sample with inhibition (4-fold dilution, where ACE is present in its inhibitor bound form (EI) and in its free form (E)) and after decreasing the inhibitor effect to a negligible level (400-fold dilution, where the concentration of ACE-inhibitor complex (EI) is negligible compared to the free enzyme (E)). Using these values the level if inhibition was estimated according to equation 3.

Human serum ACE activity was measured in hypertensive patients at 4-fold and at 400-fold dilutions. The raw enzyme activity data has been multiplied by the dilution factor (4 or 400) to calculate ACE activity in the serum. The ratio of these activities gives an estimation of the level of ACE inhibition within the sample analyzed.

**Establishing a relationship between the determined values and clinical effectiveness of the therapy**

Performing the measurements and calculation on clinical samples

Carrying out the invention we have used the values obtained from the patients who were not taking ACE inhibitor drugs. Note that the invention theoretically suggests a surprisingly robust assay to determine effectiveness regarding the untreated samples. For example, patients with no ACE inhibitor should have a very small difference in the normalized activities determined at 4-fold dilution compared to values determined at 400-fold dilution (calculated inhibition should be $0\pm$ error of the experimental method). In contrast, patients with effective ACE inhibitor therapy must have lower activities at 4-fold dilution compared to 400-fold dilution. Surprisingly, the level of inhibition was $72\pm7$ % (average $\pm$ SD) in the control group, which is much higher than the expected average of or near to 0 (Fig. 4).

Identifying human serum albumin as an endogenous inhibitor

Based on this invention this is a proof for the presence of an endogenous ACE inhibitor in the human serum. It is also a further indication of the importance and performance of the method invention, since this enormous level of endogenous inhibition has not been revealed in the past decades of intensive research leading to successful introduction of ACE inhibitor drugs into the cardiovascular practice as a cornerstone of medical therapy. Nonetheless, our data suggest that serum ACE activity is dominantly inhibited (level of inhibition $72.3\pm0.6$ %) under physiological conditions.

It was also found that ACE genotype has a limited effect on ACE activity in contrast with ACE expression. The presence of an endogenous inhibitor buffering variations in ACE amount provides an explanation to this fact, as well.

Efforts were made to identify this putative endogenous ACE inhibitor. Serum samples were ultrafiltered through different pore size filter devices. (Figure 12) No effect of filtration through a filter with 50 kDa pore size was found. In contrast, filtering through a membrane with 100 kDa pore size resulted in significantly elevated ACE activities in the 4 to 32 fold dilutions compared to the control.

In an experiment, ACE was cross-linked with interacting proteins in patient's sera and adducts comprising human serum albumin and ACE were detected (data not shown). Next, effects of HSA were tested on ACE activity. (Figure 13) HSA inhibited recombinant human ACE activity with a half maximal inhibitor concentration of 9.6 mg/ml. Important to note that maximal inhibition was 95% (partial inhibition) at 64 mg/ml HSA. (Figure 13/A) Purified serum ACE activity was also inhibited by HSA (Figure 13/B) with the IC50 value of 6.1 mg/ml. The maximal inhibition was 95% at 64 ng/ml. Finally, recombinant ACE activity was inhibited by human serum. (Figure 13/C) Dilution resulted in an increase in exogenous ACE activity, similar to results with endogenous ACE (Figure 11/B).

Thus, if the level of inhibition by HSA is to be assessed by the method of invention, the dilution factors should preferably be set as follows.

The high dilution factor in case of a sample in which the inhibitor bound form is negligible is at least 5, preferably at least 8 or at least 10 or highly preferably at least 12, 16 or 20.

The low dilution factor in case of a sample in which the active form is considered as negligible is at most 3 or at most 2.5 or highly preferably at most 2.

The ratio of dilution factors is thus at least 3 or preferably at least 4 or at least 5 or at least 8.

There were indications in the art that an endogenous inhibitor may exist but it has not been identified. Kramers et al presented a point mutation in the stalk region of the ACE gene, therefore circulating ACE amount is dramatically elevated (5-fold) [Kramers C, et al. Circulation 2001;104:1236-40.]. This mutation affects at least eight families, but these members do not either suffer from clinical abnormalities or hypertension. HSA perhaps prevents them from the possible effects of extremely high ACE amount. Nesterovitch at al published another mutation with 13-fold elevated ACE concentration in the serum, but they could not link it to any disease [Nesterovitch AB et al. PLoS One 2009;4:e8282.]. There are some other mutations of the ACE gene which are coupled with mildly elevated ACE amount, but in that cases not even cardiovascular diseases were observed [Semmler A, et al. Clin Chem Lab Med 2006;44:1088-9.; Linnebank M, et al. Neurology 2003;61:1819-20.; Eyries M et al. J Biol Chem 2001;276:5525-32.].

Serum angiotensin-converting enzyme is pathologically elevated in some granulomatous diseases (e.g. sarcoidosis) and its connection with Gaucher's disease was described previously [Lieberman J et al. N Engl J Med 1976;294:1442-4. Studdy P, et al. Lancet 1978;2:1331-4.]. In spite of their increased ACE amount, the incidence of cardiovascular diseases do not differ from that of the healthy population.

Serum albumin influences not only the circulating ACE but the membrane-bound form, too. This finding is supported by workgroups of Danser and van Dijk, who in vivo examined the angiotensin I conversion in human forearms of different genotype patients [Danser AH et al., J Hypertens 1999;17:1867-72.; van Dijk MA, et al. J Cardiovasc Pharmacol 2000;35:484-90.]. In these studies local angiotensin I to angiotensin II conversion was found to be independent from the I/D genotype, and angiotensin II/I ratio was similar in II, ID or DD patients. Previously, more workgroups proposed the existence of an endogenous ACE inhibitor, but they could not establish its nature. Lieberman et al recommended routinely the serum dilution before measurement of ACE activity to eliminate the effect of the endogenous inhibitor. They found that the inhibitor is a protein of its nature, which has a molecular weight over 50kDa [Lieberman J. and Sastre A. Chest 1986;90:869-75.].

Defining inhibitor values representing clinically effective inhibition

ACE inhibitor drugs should increase the level of serum ACE inhibition even in the presence of the endogenous inhibitor. Indeed, the estimated serum ACE inhibition in patients taking ACE inhibitor drugs was $93 \pm 8$ %, in accordance with the predictions of the description (Fig. 4). This substantial difference (72% in patients without ACE inhibitor therapy versus 93% in patients with ACE inhibitor therapy) suggests that effectiveness of ACE inhibitor therapy may even be assessed irrespectively of the presence of an endogenous inhibitor. If the data representing the individual values for the patients is plotted as a histogram, the two groups of the patients (e.g. patients with or without ACE inhibitor therapy) are distinguishable (Fig. 5) with a surprising sharpness. On Fig. 5. data. shown on Fig. 4.. have been plotted as frequency distribution (histogram). The line represents the chosen value (90%) representing the threshold for effective ACE inhibitor therapy. While this value is somewhat arbitrary, and may be lower or higher with a few percentage, this would affect only a few patients who belong to a borderline group. Otherwise the patients are clearly separated to two large groups comprising those in which the inhibition is sufficient and those in which it is not.

The frequency distribution on Fig. 5. clearly shows that endogenous inhibition in patients without ACE inhibitor therapy (n=158) is limited to the range of 46 to 87%. In contrast, the majority of patients taking ACE inhibitor drugs had higher levels of inhibition (n=383). The clinical effectiveness of serum ACE inhibition may therefore theoretically be established by the method described in the invention. We have tested this by choosing 90% inhibition as a threshold for clinically significant serum ACE inhibition (Fig. 5).

Relation with blood pressure

Next, the ACE inhibition was determined by the dilution method correlates with blood pressure (Fig. 6). ACE activity was determined at 4 and 400-fold dilutions of serum samples, besides to the measurement of blood pressure. Apparent ACE inhibition was calculated by the ratio of the values upon 400-fold and 4-fold dilutions. Mean arterial blood pressure of the patients with apparent ACE inhibition below (n=68) or above (n=318) of 90% are shown. Bars represent the mean and standard error of the mean, while asterisks represent the significant difference between the two groups (p=0.003).

The observed effect of ACE inhibitor treatment to decrease arterial blood pressure was in accordance with large clinical trials, like EUROPA (Fox, KM, et al, Lancet. 2003 Sep 6;362(9386):782-8.): we have found a decrease of 5.8 mmHg in mean arteriolar pressure, while that was 6.5 mmHg in the EUROPA trial. It is worth to mention that effectiveness of ACE inhibition was compared to the placebo group in the EUROPE trial, while our groups were based on the biochemical effectiveness of the inhibition. The similar values therefore suggest accurate identification of patients with insufficient ACE inhibition by the invention.

Consequences of ACE activation

The activation of ACE during pathophysiological states may lead to diabetes, cardiac failure, diabetes, besides other disorders. Up to date no dominant role for endogenous serum ACE inhibition has been mentioned in the progression of disease where angiotensin converting enzyme may be involved. Nonetheless, our data suggest that changes in the inhibition may contribute to the progression of diseases related to ACE. Moreover, our data strongly suggest that ACE activity may be indirectly modulated by agents affecting the interaction between ACE and the endogenous inhibitor, pinpointing this interaction as a new therapeutic target in many diseases.

Patient tailored therapy

The determined effectiveness of the inhibition may also be used to establish a personalized therapy or even optimize a therapy in terms of the apparent side effects. This process may also be used to estimate appropriate or the optimal drug dosage to avoid unwanted side effects. In this case an upper value for inhibition should be determined or the dosage may also be adjusted under the control of the determination. In the first case clinical trials should determine the level of inhibition which achieves maximal clinical effects. Dosage of the drug should be decreased in the case of patients with inhibitor level higher than the determined maximally effective inhibition. Alternatively, the dose may be decreased to a level when the patient is devoid of side effects and the level of inhibition may be determined to decide whether the tolerated dose is sufficient to inhibit the enzyme. When inhibition is found to be insufficient, then the drug should be excluded from the therapeutic regimen of the patient. On the contrary, when enzyme inhibition is still sufficient at the lower dose, then the patient's therapy is optimized.

In general, the process according to the description relates to a method for setting the dose of an inhibitor of an enzyme in a patient, wherein an upper level of inhibition is determined or a predetermined upper level of inhibition is provided, and
a lower level of inhibition is determined or a predetermined lower level of inhibition is provided, and the level of inhibition is assessed in a patient by a method of the invention and the dosage of the drug is decreased in the patient if the assessed inhibitor level is higher than the upper level of inhibition the dosage of the drug is increased in the patient if the assessed inhibitor level is lower than the lower level of inhibition. said inhibitor. Preferably, the upper level of inhibition is near to the maximally effective inhibition and the lower level of inhibition is or near to the level wherein the inhibition is ineffective.

**Estimating the level of inhibition by an exogenous enzyme**

The dilution based method can be applied to the estimation of inhibition in cases when the level of endogenous enzyme activity (low activity or enzyme is not expressed in the sample, such as HMG CoA reductase, etc.) or the sensitivity of the detection is not sufficient to measure enzyme activities at high dilution factors.

The key of the successful measurements is to add exogenous enzyme to the sample, then measure the changes in the activity of the endogenous enzyme. In the case of the presence of an inhibitor, the activity of the exogenous enzyme is inhibited at low dilution, while the uninhibited activity can be determined at high dilution as it has been described in the case of endogenous ACE.

The method was tested using exogenous ACE. Exogenous ACE was added to patient's serum samples to have at least 100-times higher activity compared to the endogenous ACE. ACE activity measurement was therefore sensitized. Serum samples of a patient on ACE inhibitor therapy were supplemented with exogenous ACE and were serially diluted and enzyme activities were measured (Fig. 7.). On Figure 7 exogenous ACE activity is shown as a function of the dilution of the exogenous ACE containing sera, thereby ACE inhibition in the sera of a patient on ACE inhibitor therapy.

The results of the determination can be evaluated according to equation 3: enzyme activity was 0.88 AU/ml and 102.4 AU/ml at dilutions of 2-fold and 128-fold, respectively. These data suggest that the inhibitor concentration of the serum was sufficient to inhibit exogenous ACE at a level of 99.1%.

It needs to be noted that the experiment can be performed to estimate inhibitor content within an arbitrary solution using the appropriate exogenous enzyme. The method can therefore be applied in a wide range of potential situations, such as the estimation of the concentration of a drug acting on an enzyme within the central nervous system in sera, or cerebrospinal fluid. The process according to the description can also be employed to estimate inhibitor content during the chemical or microbiological production of drugs, or to estimate inhibitor content in natural fluid. Moreover, the present technology may also been applied to determine the actual augmentation of biological, biochemical or chemical reactions by reversible activators (such as cofactors, catalysts, etc).

Validation of ACE activity measurement

The circulating ACE amount is genetically determined. Patients, who are homozygote for the allele D of the ACE gene had 56% higher ACE concentration in their serum than those who are homozygote for the allele I (II= $101 \pm 7$ ng/ml, n= 28; ID=$115 \pm 5$ ng/ml, n= 70; DD= $158 \pm 6$ ng/ml serum, n= 56). (Figure 10.) ACE activity was also elevated in DD homozygote patients, although the degree of elevation, measured in 4-fold diluted serum (II= $32 \pm 2$ U/ml, n= 28; ID= $37 \pm 1$ U/ml, n= 70; DD= $44 \pm 1$ U/ml, n= 56), was lower than expected.

This controversy may be explained by methodological factors. To validate the ACE activity measurement, FAPGG cleavage (photometric determination) was compared to the angiotensin I conversion (determined by HPLC). ACE activity determining by FAPGG cleavage was directly proportional to angiotensin I to angiotensin II conversion.

(Figure 11/A) Nonetheless FAPGG cleavage was about 20-fold faster and easier to measure. Another important factor is the linearity of the method. Indeed, when purified tissue ACE was tested, we found no effects of dilution on the determined ACE activity. However, serum ACE activity depended on the dilution. (Figure 11/B) In particular, a biphasic effect of dilution was found on ACE activity, an initial increase in activity (up to 16-fold dilution) was followed by a plateau (16-64-fold dilutions) and by a further increase (more than 64-fold dilutions). (Figure 11/B) These data suggested the presence of a soluble endogenous ACE inhibitor. When the serum is undiluted, the inhibitor binds to ACE, masking its activity (inhibiting), and when the serum is diluted, the inhibitor dissociate from the ACE, unmasking its activity. (Figure 11/C)

## Methods

### 1.1 Blood sample collection, serum and DNA preparation

Blood samples were collected from volunteers using standard aseptic technique. Native blood was incubated for 60 minutes at room temperature, serum fractions (separated by centrifugation 1500g, 15 min) were kept at -25 °C until further experiments. Genomic DNA was prepared from anticoagulated venous blood using a DNA separation kit (FlexiGene DNA kit, Cat. No. 51204; Qiagen GmbH).

### 1.2 ACE activity measurement

ACE activity was determined at 37°C using an artificial substrate of ACE (FAPGG, (*N*-[3-(2-furyl)acryloyl]-L-phenylalanylglycylglycine; Sigma-Aldrich; Cat. No. F7131). The reaction mixture contained: 25 mM HEPES (N-2-hydroxyethylpiperazine-N-2-ethanesulfonic acid), 0.5 mM FAPGG, 300 mM NaCl and serum, the pH was 8.20. The measurement was performed in 96-well plates (Greiner-Bio One: Cat. No.: 655101) using a plate reader (NovoStar plate reader, BMG Labtech GmbH). Changes is absorbance (340 nm) were measured at each 5 min for at least 90 min. Optical density values were plotted as a function of reaction time and fitted with linear regression. ACE activity was calculated by the equation:

$$\text{Activity} = -(S/k)*D \qquad (4)$$

where S: slope of the decrease in optical density by time, k: change of optical density upon cleavage of 1 nmol FAPGG, D: dilution of the sera. ACE activity is given in units, where 1U equals to cleavage of 1nmol FAPGG in one minute. In some experiments the reaction mixture also contained human serum albumin (HSA, Cat. No.: N1-201000-211, Human BioPlazma Manufacturing and Trading LTD).

### 1.3 Determination of ACE genotype (I/D polymorphism)

The insertion/deletion genotype of ACE was examined by PCR amplification of alleles I and D on the basis of a standard protocol described by Rigat et al. [Rigat B, Hubert C, Alhenc-Gelas F, Cambien F, Corvol P, Soubrier F. An insertion/deletion polymorphism in the angiotensin I-converting enzyme gene accounting for half the variance of serum enzyme levels. J Clin Invest 1990;86:1343-6.].The amplification products were separated using 5% polyacrylamide gel electrophoresis, and were visualised by ethidium-bromide staining. The presence of the allele I and D resulted in a 490-bp and a 190-bp PCR product, respectively.

### 1.4 Serum ultrafiltration

Serum sample from a healthy volunteer was ultrafiltered after a 250-fold dilution in 25 mM HEPES pH 8.20 by a 50 and 100 kDa pore size ultrafiltration device (Vivaspin 500, Cat. No.: VS0231, VS0241; Sartorius Stedim Biotech) at 4 °C for 6 minutes at 15000 g. Ultrafiltration was repeated until the diluted samples were reconcentrated to the initial volume.

### 1.5 HPLC analysis of angiotensin I conversion

Serum samples were incubated at 37 °C containing 0.5 $\mu$M angiotensin I (synthetised by GenScript USA Inc.) and 300 mM NaCl in 25 mM HEPES buffer, pH 8.20. 5 mM EDTA was added to stop the reaction. Angiotensin peptides were measured after filtering through 10 kDa pore size filter (Vivaspin 500, Cat. No.: VS0101; Sartorius Stedim Biotech). HPLC analysis was performed using Shimadzu HPLC with a reverse phase C18 column (Hypersil Gold, Cat. No.:

25005-154630; Thermo Scientific). Eluent A contained 0.01% trifluoroacetic acid (TFA, Sigma-Aldrich; Cat. No.302031) in water, while eluent B contained 0.01% TFA in acetonitrile (Sigma-Aldrich, Cat. No.34998). Angiotensin peptides were separated using a gradient elution profile from 22% acetonitrile to 55% acetonitrile content. Angiotensin peptides were detected by a diode array detector at 230 nm and the area under the curve of each angiotensin peptide peeks were compared to calibration curves. The amount of angiotensin peptides were plotted against the time of reaction and fitted by a linear regression. The kinetics of angiotensin I conversion is given in nmol angiotensin I cleavage in 1 ml of serum in one minute.

## 1.6 ACE amount measurement

Serum ACE amount was measured by using a commercial human ACE ELISA development kit (R&D Systems Inc., Cat. No.DY929) according to the manufacturer's instructions with minor modification. Briefly, enzyme-linked immunosorbent plates were coated with 80 ng/well capture antibody, then the remaining binding sites were blocked with reagent diluent (10 mg/ml bovine serum albumin (Cat) in Dulbecco phosphate-buffered saline solution (Cat)). 100-fold diluted sera (in reagent diluent) were added to the wells, then the antibody-antigen complexes were labelled with a biotinylated detection antibody (20 ng/well). 200-fold diluted streptavidine conjugated to horseradish-peroxidase (kit component) was added to the wells. Finally, the amount of complexes were detected with substrate solution (0.3 mg/ml tetramethylbenzidine, 0.1 $\mu$M $H_2O_2$, 50 mM acetic acid), the reaction was terminated after 20 minutes with 0.5 mmol/L HCl, and the optical density was measured at 450nm. The samples were measured at least three times or more until we reached a value of standard deviation less than 15%.

## 1.7 Serum crosslinking

The ACE - HSA interaction was stabilized with heterobifunctional crosslinkers. The initial mixture was incubated for 1 hour at 25°C and contained 4-fold diluted serum, 1,25mM (+) or 2,5mM (++) or 5mM (+++) succinimidyl-[(N-maleimidopropionamido)-dodecaethyleneglycol] ester (SM(PEG)$_{12}$, Thermo Scientific, Cat No.22112), and succinimidyl-[(N-maleimidopropionamido)-hexaethyleneglycol] ester (SM(PEG)$_6$, Thermo Scientific, Cat. No.22105) in 25mM HEPES, pH 7.2. Remaining active crosslinker molecules were blocked by using 50mM tris(hydroxymethil)aminomethane (TRIS, Sigma-Aldrich, Cat. No.T1503) for 30 minutes at room temperature. Biotinylated goat anti-human ACE antibody (R&D Systems Inc. Part No.841366, 22.6ng/ml) and immobilised streptavidin resin (Pierce, Cat. No.: 20349) were added to the mixture to anchor the crosslinked products to the surface of the resin. The blank contained 22.6ng/ml non-specific goat IgG instead of ACE specific antibody. After incubation overnight at room temperature the resin was purified by washing five-times using 25mM HEPES pH 7.20. Purified proteins were removed from the resin by boiling for 10 minutes in 2x concentrated SDS sample buffer (Sigma-Aldrich, Cat No.S3401). The crosslinked proteins were visualised by Western-blot analysis after gel electrophoresis in a 5-15% gradient gel (Mini-Protean TGX Precast Gel, Cat. No.456-1086, Bio-Rad Laboratories Inc.) The primary antibody was a goat anti-human ACE antibody used in 1:1000 dilution (Part. No. 841365; R&D Systems Inc.), the secondary antibody was a peroxidase-linked anti-goat antibody used in 1:40 000 dilution. The blot was developed on green sensitive medical X-ray film (Primax Berlin GmbH, Cat. No. ?) after adding Western Lightning Plus-ECL reagent (Perkin Elmer life sciences, Cat. No.NEL10400).

### 1.7.1 Ultrafiltered serum and HSA crosslinking

The previous experiment was performed with HSA and 100kDa pore size ultrafiltered serum instead of whole serum. The initial mixture contained 3.5 fold diluted filtered serum, 0mg/ml (+), 24mg/ml (++) or 48mg/ml (+++) HSA and 5mM SM(PEG)$_6$ in 25mM HEPES buffer pH 7.2. The following steps were the same as described above. After the developing the Western-blot, the membrane was stripped for 20 minutes at 60°C in stripping buffer containing 1% dodecylsulfate-sodium salt (SDS, Serva, Cat. No.20765), 20mM DL-dithiotreitol (DTT, Sigma-Aldrich, Cat. No.D9779) in 62.5mM Tris buffer, pH 7.5. The visualisation protocol was repeated using biotinylated anti-human serum albumin antibody (Exbio Praha, Cat. No.1B-257-C100) as primary antibody in 1:5000 dilution, and peroxidase-streptavidin in 1:100 000 dilution (Jackson, Cat. No.016-030-084) to link peroxidase to the complex.

### 1.7.2 Recombinant ACE and HSA crosslinking on the surface of ELISA plate

Recombinant ACE-HSA interaction was examined on ELISA plate (Greiner Bio-One; Cat. No.655061). 300$\mu$l 1% HSA in phosphate buffer saline solution (PBS pH 7.2 containing KCl 2.7mM, $KH_2PO_4$ 1.5mM, NaCl 137mM, $Na_2HPO_4$ 8mM) was added to the wells and incubated overnight at room temperature to allow surface binding. After washing with PBS, 100$\mu$l of 625$\mu$M SM(PEG)$_6$ and SM(PEG)$_{12}$ were added to the wells and incubated for 30 minutes. After thorough washing, 100$\mu$l of 260ng/ml recombinant ACE was added to the wells (R&D Systems, Part No.841367) for 1 hour. 100$\mu$l

of biotinylated goat anti-human ACE antibody (R&D Systems Inc. Part No.841366, 22.6ng/ml) was added to the plate after a 2-hour-washing step. Then 100µl of streptavidine conjugated to horseradish-peroxidase (R&D Systems, Part No.890803) was added for 30 minutes to each well followed by washing. Finally, the bound ACE - detection antibody - streptavidine-HRP complex was visualised by a substrate solution (containing 80 mM acetic-acid, 0.4 mg/ml tetrame-thylbenzidine, 0.56 µM $H_2O_2$) in plate reader at 380nm. As controls, the crosslinker was blocked with 100µl of 330 mM Tris-HCl solution (column 2) or left alone (column 3). Non-specific binding between proteins was tested with 1% gelatine solution in the presence (column 5) or in the absence (column 4) of crosslinker-blocking. As positive control, a sandwich ELISA was performed with the reagents of commercial human ACE ELISA development kit. The measurement was performed in triplicates in each time-point.

### 1.8 Recombinant ACE

Recombinant ACE was produced by a Bac-to-Bac TOPO expression system (Invitrogen, Cat. No.A11101) according to the manufacturer's instructions. Briefly, chemically competent E. coli strain (Invitrogen, Cat. No.C8620-03) was transformed with ACE gene containing cDNA plasmid (GeneCopoeia; Cat. No. EX-T7349-101). After antibiotic selection and plasmid isolation the pFastBac construct containing ACE gene was transformed into DH10Bac competent E. coli (Invitrogen; Cat. No.10361-012) to generate recombinant bacmid. The bacmid DNA was transfected into the SF9 insect cell line (Invitrogen, Cat. No.10362-100), in which baculovirus was generated. Further SF9 insect cells were infected by these bacoluviruses. At day 4 the insect cell was (1000 g, 10 min, 4 °C) and the pellets were washed in PBS to eliminate cell culture medium. The pellet was homogenised in RIPA buffer (50 mM tris, 150 mM NaCl, 1% Triton X 100, 0.1% SDS, 1% deoxi-cholate) by a sonicator (type). The supernatant was collected (15000 g, 10 min, 4 °C), and it was injected in 25 mM HEPES, 15 mM NaCl, pH 8.2 onto a anion exchange column (Knauer, Biofox 40 µm Q anion exchange column, cat. no.Y4011). ACE was eluated with a gradually increasing concentration of NaCl (from 168 mM to 540 mM), the ACE peek was determined by ACE activity measurement of the collected fractions.

### 1.9 ACE activity assay

The assay was performed in a solution containing sera (different dilutions), 0.1 mM substrate (FAPGG, N- [3-(2-Furil)akryloyl]-L-fenilalayil-glycil-glycin), 300 mM NaCl and 25 mM HEPES (4-(2- Hidroxietil)piperazin-1-etánszulfonsav), pH=8.2. The measurements were performed in a 96 well microplate (Greiner Bio-One®) in 200 or 300 µl final volume. The decrease in optical densities at 340 nm was measured by a NOVOstar® plate reader at 37 oC. The decrease in optical densities were fitted by a linear regression (values were accepted when R2>0.9). ACE activity was calculated according to equation 5:

$$\text{Activity} = \alpha/(\text{OD0-0,133}) * 1093,792 * D \qquad (5)$$

where $\alpha$ is the slope of the decrease in optical density; OD0 is the initial optical density; 0,133 is the density of the measurement buffer alone (containing 25 mM HEPES and 300 mM NaCl; 1093,792 is the decrease in optical density after full decomposition of the substrate.; D is the dilution factor.1.10 Statistical analysis Baseline characteristics were compared using the chi-squared test or Fisher's exact test for qualitative variables, and Student's t test or Mann-Whitney's U test for quantitative variables. The statistical package SPSS 15.0 (SPSS Inc., Chicago, IL, USA) was used and p<0.05 was considered statistically significant.

## PRODUCT DEVELOPMENT

### Apparatus according to the description

The apparatus of the description comprises a sample receiving unit suitable to receive at least two samples, preferably at least two identical samples. Thus the sample receiving unit has multiple sample containers. The sample containers are formed so that they are suitable for receiving samples of different volumes.

The apparatus comprises a diluting unit which is suitable to lead various reagents to the sample container. Appropriate mixing upon dilution is to be ensured. This can be achieved by an appropriate arrangement of the dispenser and the form of the container. Preferably the container has a rounded shape so as to avoid non-mixable spaces.

A specific feature of the apparatus is that the apparatus has a detection system which is suitable to detect and monitor enzymatic reactions thereby measuring enzyme activity. The detection system can be a spectrophotometric detection system comprising e.g. a light source and a detector, or can be a fluorescent detection system or an UV-VIS detection system depending on the reaction compounds and/or products of the enzyme reaction or any other means which can be used to determine enzymatic activity. In the detection system preferably the conversion of an enzyme substrate is followed by detecting either the substrate or the reaction product.

A preferred variant is a plate reader preferably combined with one or more dispenser unit driven by a set of pumps to move the diluting fluid and/or reaction constituents which are preferably buffered.

In a preferred embodiment the sample container is a cuvette. In this sense the well of a plate is considered as a cuvette in a plate reader. In a preferred embodiment, however, the cuvettes have different volumes whereas the ratio of the length of the light path cuvettes of different volume is smaller than the ratio of their volume. Preferably the lengths of the light paths are comparable or essentially identical whereas their volume is largely different.

The apparatus is preferably equipped with a data processing unit or processor and a data storing unit. These units are operably connected with the apparatus of the description. The data storing unit comprises a program suitable for

- driving the diluting unit to appropriately dilute the samples, and/or
- obtaining data from the detector unit, and/or
- and or carrying out any calculation as defined in the method of the description.

Thus desirably the apparatus is an automated apparatus.

Preferably the process according to the description relates to reagents or constituents selected from enzyme substrates, enzymes, their inhibitors, dilution buffers, reaction buffers. Preferably the constituents are in the form of a kit, preferably together with the apparatus or a suitable part thereof, e.g. cuvette or data carrier comprising the program.

In a preferred embodiment the samples or conduits transferring the samples are labeled e.g. by bar codes, and the apparatus is equipped by a label reader operably connected with the processing unit. Thereby the apparatus can automatically define the dilution factor and the inhibition levels.

In case of ACE inhibitors the invention is ready to be reduced into practice. This The skilled person is able, however, to validate the method for further targets, i.e. enzyme inhibitor pairs as well. In this case the method, first of all the detection means, shall be optimized.

Further development may aim at therapies of cardiovascular patients, specifically for ACE, HMG-CoA reductase, rennin and/or dipeptidyl peptidase-4 may serve as targets. A further area of development may be the enzyme inhibitors used in psychiatric or psychosomatic disorders.

## SUMMARY OF THE RESULTS

Applicability of the method of the process according to the description has been validated on ACE inhibitor therapy. A cohort of 501 patients with high blood pressure participated in the study, among which ACEi medication was prescribed to 387 patients. The method of the invention was applied in these patients and a successful inhibition was found in case of 82% of the 387 patients. In case of patients with insufficient inhibition we attempted to explore the reasons. The patients were reached and the importance of taking the ACEi medicament was emphasized to them. They were informed that according to our results the effectiveness of the therapy is insufficient and called back for a further control. The 45% of the patients did not come back. Raising the dose of the ACEi resulted in a successful therapy in case of 9 percent of the patients. In case of 17% of the patients the second control showed a sufficient inhibition whereas a further 17% of the patients we changed to another medicament to due to complaints regarding side effects. In 12% of the patients we could not find a reason of the insufficient therapy; in such cases we suggest to modify the therapy based on ACEi.

Blood pressure of the patients strictly correlated with successful inhibition (Figure 9).

## INDUSTRIAL APPLICABILITY

The present process according to the description allows to increase effectiveness of therapies based on enzyme inhibitor medicaments. Inappropriate therapies put a huge burden on the social care system and therefore the economical significance of a personalized approach in medicine increases. A first step in this approach is the assessment of the effectiveness of on-going or planned therapies. The present invention provides a major contribution to this field by providing a simple, potentially single step, single sample method to assess the effectiveness of ACEi therapy. Moreover, the invention provides a possibility to set or optimize a therapeutic regime without exposing the patient to excessive burden thereby improving patient compliance.

More generally, the process according to the description is suitable to effectively and simply monitor the presence of an inhibitor or the level of inhibition of any sample wherein once the method is established no further samples and no controls are required. Moreover the method of the process according to the description can be performed with reagents, e.g. substrates, normally used for a given enzyme. While automation is possible, the method typically can be carried out without expensive and overly specific equipment.

REFERENCES

Cambien F, Poirier O, Lecerf L, et al. Deletion polymorphism in the gene for angiotensin-converting enzyme is a potent risk factor for myocardial infarction. Nature 1992;359:641-4.

Cohn JN, Johnson G, Ziesche S, et al. A comparison of enalapril with hydralazine-isosorbide dinitrate in the treatment of chronic congestive heart failure. N Engl J Med 1991;325:303-10.

Corvol P, Michaud A, Soubrier F, Williams TA. Recent advances in knowledge of the structure and function of the angiotensin I converting enzyme. J Hypertens Suppl 1995;13:S3-10.

Cuspidi C, Muiesan ML, Valagussa L, et al. Comparative effects of candesartan and enalapril on left ventricular hypertrophy in patients with essential hypertension: the candesartan assessment in the treatment of cardiac hypertrophy (CATCH) study. J Hypertens 2002;20:2293-300.

Danser AH, Deinum J, Osterop AP, Admiraal PJ, Schalekamp MA. Angiotensin I to angiotensin II conversion in the human forearm and leg. Effect of the angiotensin converting enzyme gene insertion/deletion polymorphism. J Hypertens 1999;17:1867-72.

Eyries M, Michaud A, Deinum J, et al. Increased shedding of angiotensin-converting enzyme by a mutation identified in the stalk region. J Biol Chem 2001;276:5525-32.

Fox KM. Efficacy of perindopril in reduction of cardiovascular events among patients with stable coronary artery disease: randomised, double-blind, placebo-controlled, multicentre trial (the EUROPA study). Lancet 2003;362:782-8.

Fyhrquist F. Tikkanen I., Gronhagen-Riska C., Hortling L., Hichens M., "Inhibitor Binding Assay for Angiotensin-Converting Enzyme" Clin Chemistry, 1984 30(5) 696-700.

Greenberg B, Quinones MA, Koilpillai C, et al. Effects of long-term enalapril therapy on cardiac structure and function in patients with left ventricular dysfunction. Results of the SOLVD echocardiography substudy. Circulation 1995;91:2573-81.

Gronhagen-Riska C., Tikkanen I., Fyhrquist F., "Competitive inhibitor binding assay (CIBA) of ACE inhibitors", Acta. Med. Scand. Suppl., 1986, 714, 43-47.

Hoogwerf BJ, Young JB. The HOPE study. Ramipril lowered cardiovascular risk, but vitamin E did not. Cleve Clin J Med 2000;67:287-93.

Hubert C, Houot AM, Corvol P, Soubrier F. Structure of the angiotensin I-converting enzyme gene. Two alternate promoters correspond to evolutionary steps of a duplicated gene. J Biol Chem 1991;266:15377-83. ISIS-4: a randomised factorial trial assessing early oral captopril, oral mononitrate, and intravenous magnesium sulphate in 58,050 patients with suspected acute myocardial infarction. ISIS-4 (Fourth International Study of Infarct Survival) Collaborative Group. Lancet 1995;345:669-85.

Kramers C, Danilov SM, Deinum J, et al. Point mutation in the stalk of angiotensin-converting enzyme causes a dramatic increase in serum angiotensin-converting enzyme but no cardiovascular disease. Circulation 2001;104:1236-40.

Lieberman J, Beutler E. Elevation of serum angiotensin-converting enzyme in Gaucher's disease. N Engl J Med 1976;294:1442-4.

Lieberman J, Sastre A. An angiotensin-converting enzyme (ACE) inhibitor in human serum. Increased sensitivity of the serum ACE assay for detecting active sarcoidosis. Chest 1986;90:869-75.

Lindpaintner K, Pfeffer MA, Kreutz R, et al. A prospective evaluation of an angiotensin-converting-enzyme gene polymorphism and the risk of ischemic heart disease. N Engl J Med 1995;332:706-11.

Linnebank M, Kesper K, Jeub M, et al. Hereditary elevation of angiotensin converting enzyme suggesting neurosarcoidosis. Neurology 2003;61:1819-20.

Luders S, Schrader J, Berger J, et al. The PHARAO study: prevention of hypertension with the angiotensin-converting enzyme inhibitor ramipril in patients with high-normal blood pressure: a prospective, randomized, controlled prevention trial of the German Hypertension League. J Hypertens 2008;26:1487-96.

Nesterovitch AB, Hogarth KD, Adarichev VA, et al. Angiotensin I-converting enzyme mutation (Trp1197Stop) causes a dramatic increase in blood ACE. PLoS One 2009;4:e8282.

Oppong SY, Hooper NM. Characterization of a secretase activity which releases angiotensin-converting enzyme from the membrane. Biochem J 1993;292 (Pt 2):597-603.

Pfeffer MA, Braunwald E, Moye LA, et al. Effect of captopril on mortality and morbidity in patients with left ventricular dysfunction after myocardial infarction. Results of the survival and ventricular enlargement trial. The SAVE Investigators. N Engl J Med 1992;327:669-77.

Rigat B, Hubert C, Alhenc-Gelas F, Cambien F, Corvol P, Soubrier F. An insertion/deletion polymorphism in the angiotensin I-converting enzyme gene accounting for half the variance of serum enzyme levels. J Clin Invest 1990;86:1343-6.

Ruggenenti P, Fassi A, Ilieva AP, et al. Preventing microalbuminuria in type 2 diabetes. N Engl J Med 2004;351:1941-51.

Semmler A, Stein RW, Caplan L, Danilov SM, Klockgether T, Linnebank M. Hereditary hyper-ACE-emia due to the Pro1199Leu mutation of somatic ACE as a potential pitfall in diagnosis: a first family outside Europe. Clin Chem Lab Med 2006;44:1088-9.

Six-month effects of early treatment with lisinopril and transdermal glyceryl trinitrate singly and together withdrawn six weeks after acute myocardial infarction: the GISSI-3 trial. Gruppo Italiano per lo Studio della Sopravvivenza nell'Infarto Miocardico. J Am Coll Cardiol 1996;27:337-44.

Staessen JA, Wang JG, Ginocchio G, et al. The deletion/insertion polymorphism of the angiotensin converting enzyme gene and cardiovascular-renal risk. J Hypertens 1997;15:1579-92.

Studdy P, Bird R, James DG. Serum angiotensin-converting enzyme (SACE) in sarcoidosis and other granulomatous disorders. Lancet 1978;2:1331-4.

van Dijk MA, Kroon I, Kamper AM, Boomsma F, Danser AH, Chang PC. The angiotensin-converting enzyme gene polymorphism and responses to angiotensins and bradykinin in the human forearm. J Cardiovasc Pharmacol 2000;35:484-90.

Wei L, Alhenc-Gelas F, Soubrier F, Michaud A, Corvol P, Clauser E. Expression and characterization of recombinant human angiotensin I-converting enzyme. Evidence for a C-terminal transmembrane anchor and for a proteolytic processing of the secreted recombinant and plasma enzymes. J Biol Chem 1991;266:5540-6.

## Claims

1. An *in vitro* process to assess the effectiveness of enzyme inhibition in angiotensin converting enzyme (ACE) inhibitor therapy in a subject comprising:

   (a) in a blood sample or a blood derived sample provided from a subject treated with an ACE inhibitor;
   (b) taking at least two aliquots from the sample;
   (c) diluting said aliquots by different dilution factors,
   (d) measuring the ACE activity in the dilution samples;
   (e) defining a high dilution factor where the effect of the ACE inhibitor becomes negligible, wherein said dilution factor is at least 50, preferably at least 100;
   (f) defining a low reference dilution factor, said dilution factor being at most 10;
   (g) multiplying the measured activity values in each of the diluted samples by the respective dilution factor to obtain calculated ACE activity values for each dilution samples;
   (h) obtaining the level of ACE inhibition in the original sample by comparing the enzyme activity values determined at said low and high dilution factors in said dilution samples;
   (i) assessing the effectiveness of medical treatment by the ACE inhibitor in said subject by evaluating the calculated level of ACE inhibition, wherein a low enzyme inhibition is an indication of an ineffective therapy, and wherein a high enzyme inhibition is an indication of an effective therapy.

2. The process according to claim 1 wherein the high dilution factor, wherein the effect of the ACE inhibitor is negligible, is at least 100 or at least 200, preferably at least 300 or at least 350 or highly preferably at least 400, and wherein the reference low dilution factor is at most 20 or at most 10 or highly preferably at most 5 or at most 4, and/or the ratio of dilution factors is thus at least 10 or preferably at least 30 or at least 70 or at least 100.

3. The process of claim 1, wherein in step (i) enzyme inhibition is considered as insufficient in a subject having a level of inhibition, given in percentage, lower than 90% and effective in a subject having a level of inhibition of at least 90%, thereby assessing the effectiveness of said ACE inhibitor therapy in said subject.

## Patentansprüche

1. Ein *in vitro* Verfahren zur Feststellung der Wirksamkeit der Enzymhemmung in Angiotensinkonvertierendes Enzym (ACE) Hemmungstherapie in einem Individuum, enthaltend:

   (a) in einer Blutprobe oder einer vom Blut abgeleiteten Probe erhalten von einem mit ACE-Hemmer behandelten Individuum;

(b) Entnehmen von mindestens zwei Aliquoten aus der Probe;

(c) Verdünnung der Aliquoten mit unterschiedlichen Verdünnungsfaktoren,

(d) Messung der ACE-Aktivität in den verdünnten Proben;

(e) Bestimmung eines hohen Verdünnungsfaktors, wobei die Wirkung des ACE-Hemmers vernachlässigbar wird, wobei der Verdünnungsfaktor mindestens 50, bevorzugt mindestens 100 beträgt;

(f) Bestimmung eines niedrigen Referenzverdünnungsfaktors, welcher höchstens 10 ist;

(g) Multiplizieren der gemessenen Aktivitätswerte in jeder verdünnten Probe mit dem jeweiligen Verdünnungsfaktor, um berechnete ACE-Aktivitätswerte für jede verdünnte Probe zu erhalten;

(h) Erhalten des Wertes der ACE-Hemmung in der ursprünglichen Probe durch Vergleichung der Enzymaktivitätswerte, welche in den verdünnten Proben bei den niedrigen und hohen Verdünnungsfaktoren bestimmt wurden;

(i) Feststellung der Wirksamkeit der medizinischen Behandlung mit dem ACE-Hemmer in dem Individuum durch Bewertung des berechneten Wertes der ACE-Hemmung, wobei eine niedrige Enzym-Hemmung ein Indikator für eine wirkungslose Therapie ist, und wobei eine hohe Enzym-Hemmung ein Indikator für eine wirkungsvolle Therapie ist.

2. Das Verfahren nach Anspruch 1, worin der hohe Verdünnungsfaktor, bei dem die Wirkung des ACE-Hemmers vernachlässigbar ist, mindestens 100 oder mindestens 200, bevorzugt mindestens 300 oder mindestens 350 oder insbesondere mindestens 400 beträgt,

und worin der niedrige Referenzverdünnungsfaktor höchstens 20 oder höchstens 10 oder insbesondere höchstens 5 oder höchstens 4 beträgt, und/oder

das Verhältnis der Verdünnungsfaktoren also mindestens 10 oder bevorzugt mindestens 30 oder mindestens 70 oder mindestens 100 beträgt.

3. Das Verfahren nach Anspruch 1, worin im Schritt (i) in einem Individuum die Enzymhemmung mit einem Hemmungswert, gemessen in Prozent, niedriger als 90% als unwirksam und in einem Individuum mit einem Hemmungswert von mindestens 90 % als wirksam betrachtet wird,

um die Wirksamkeit der ACE-Hemmungstherapie in dem Individuum festzustellen.


**Revendications**

1. Procédé *in vitro* pour évaluer l'efficacité de l'inhibition enzymatique dans la thérapie par inhibiteur de l'enzyme de conversion de l'angiotensine (ECA) chez un sujet, comprenant :

(a) dans un échantillon de sang ou un échantillon dérivé du sang provenant d'un sujet traité avec un inhibiteur de l'ECA ;

(b) prendre au moins deux parties aliquotes de l'échantillon ;

(c) diluer lesdites aliquotes par différents facteurs de dilution ;

(d) mesurer l'activité ECA dans les échantillons de dilution ;

(e) définir un facteur de dilution élevé où l'effet de l'inhibiteur de l'ECA devient négligeable, ledit facteur de dilution étant d'au moins 50, de préférence d'au moins 100 ;

(f) définir un faible facteur de dilution de référence, ledit facteur de dilution étant au plus égal à 10 ;

(g) multiplier les valeurs d'activité mesurées dans chacun des échantillons dilués par le facteur de dilution respectif pour obtenir les valeurs d'activité ECA calculées pour chaque échantillon de dilution ;

(h) obtenir le niveau d'inhibition de l'ECA dans l'échantillon original en comparant les valeurs d'activité enzymatique déterminées auxdits facteurs de dilution faible et élevé dans lesdits échantillons de dilution ;

(i) évaluer l'efficacité d'un traitement médical par l'inhibiteur de l'ECA chez ledit sujet en évaluant le niveau calculé d'inhibition de l'ECA, où une faible inhibition de l'enzyme est une indication d'un traitement inefficace et où une forte inhibition de l'enzyme est une indication d'un traitement efficace.

2. Procédé selon la revendication 1, où le facteur de dilution élevé, où l'effet de l'inhibiteur de l'ECA est négligeable, est d'au moins 100 ou d'au moins 200, de préférence d'au moins 300 ou d'au moins 350 ou très préférentiellement d'au moins 400,

et où le facteur de faible dilution de référence est d'au plus 20 ou d'au plus 10 ou très préférentiellement d'au plus 5 ou d'au plus 4, et/ou

le rapport des facteurs de dilution est donc d'au moins 10 ou de préférence d'au moins 30 ou d'au moins 70 ou d'au moins 100.

3. Procédé selon la revendication 1, où à l'étape (i), l'inhibition de l'enzyme est considérée comme insuffisante chez un sujet présentant un niveau d'inhibition, exprimé en pourcentage, inférieur à 90% et efficace chez un sujet présentant un niveau d'inhibition d'au moins 90%,
évaluant ainsi l'efficacité de ladite thérapie par inhibiteur de l'ECA chez ledit sujet.

Figure 1. Dose response for captopril on human serum angiotensin converting enzyme (ACE) activity.

Figure 2  Effect of dilution on the activity of inhibited recombinant ACE.

**Figure 3. Theoretical effect of serum dilution on the inhibition of human ACE by captopril.**

**Figure 4. Calculated serum ACE inhibition in hypertensive patients.**

**Figure 5. Different levels of serum ACE inhibition in patients.**

**Figure 6. ACE inhibition determined by the dilution method correlates with blood pressure.**

**Figure 7. Increase in exogenous ACE activity upon dilution of the sera.**

**Figure 8 Differences in concepts of determination of enzymatic activities**

**Figure 9 Validation of the clinical effectiveness of described invention**

**Figure 10  Inconsistency in ACE expression and activity**

Figure 11 Indications of an endogenous inhibitor

Figure 12 Molecular mass of the endogenous inhibitor of ACE in the serum

**Figure 13  Inhibition of serum ACE by serum albumin**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040081957 A1
- WO 2001085985 A1
- US 6090570 A
- WO 2003025561 A1
- US 2001045643 W
- EP 02728295 A

### Non-patent literature cited in the description

- **FYHRQUIST F. et al.** Inhibitor Binding Assay for Angiotensin-Converting Enzyme. *Clin Chemistry,* 1984, vol. 30 (5), 696-700
- **GRÖNHAGEN-RISKA C. et al.** Competitive inhibitor binding assay (CIBA) of ACE inhibitors. *Acta. Med. Scand. Suppl.,* 1986, vol. 714, 43-47
- Enzyme and transporter based drug-drug interactions. Springer, 2010
- **LIEBERMAN J. ; SASTRE A.** *Chest,* 1986, vol. 90, 869-75
- **CORVOL P ; MICHAUD A ; SOUBRIER F ; WILLIAMS TA.** Recent advances in knowledge of the structure and function of the angiotensin I converting enzyme. *J Hypertens Suppl,* 1995, vol. 13, 3-10
- **HUBERT C et al.** *J Biol Chem,* 1991, vol. 266, 15377-83
- **OPPONG SY et al.** *Biochem J,* 1993, vol. 292, 597-603
- **WEI L et al.** *J Biol Chem,* 1991, vol. 266, 5540-6
- **PFEFFER MA et al.** *N Engl J Med,* 1992, vol. 327, 669-77
- **FOX KM.** *Lancet,* 2003, vol. 362, 782-8
- *J Am Coll Cardiol,* 1996, vol. 27, 337-44
- *Lancet,* 1995, vol. 345, 669-85
- **COHN JN et al.** *N Engl J Med,* 1991, vol. 325, 303-10
- **GREENBERG B et al.** *Circulation,* 1995, vol. 91, 2573-81
- **LUDERS S et al.** *J Hypertens,* 2008, vol. 26, 1487-96
- **CUSPIDI C et al.** *J Hypertens,* 2002, vol. 20, 2293-300
- **RUGGENENTI P.** *N Engl J Med,* 2004, vol. 351, 1941-51
- **HOOGWERF BJ et al.** *Cleve Clin J Med,* 2000, vol. 67, 287-93
- **RIGAT B et al.** *J Clin Invest,* 1990, vol. 86, 1343-6
- **CAMBIEN F et al.** *Nature,* 1992, vol. 359, 641-4
- **STAESSEN JA et al.** *J Hypertens,* 1997, vol. 15, 1579-92
- **LINDPAINTNER K. et al.** *N Engl J Med,* 1995, vol. 332, 706-11
- **SIMONETTA RONCA-TESTONI.** *Clinical Chemistry,* 1986, vol. 29 (6), 1093-1096
- **KRAMERS C et al.** *Circulation,* 2001, vol. 104, 1236-40
- **SEMMLER A et al.** *Clin Chem Lab Med,* 2006, vol. 44, 1088-9
- **LINNEBANK M et al.** *Neurology,* 2003, vol. 61, 1819-20
- **EYRIES M et al.** *J Biol Chem,* 2001, vol. 276, 5525-32
- **LIEBERMAN J et al.** *N Engl J Med,* 1976, vol. 294, 1442-4
- **STUDDY P et al.** *Lancet,* 1978, vol. 2, 1331-4
- **DANSER AH et al.** *J Hypertens,* 1999, vol. 17, 1867-72
- **VAN DIJK MA et al.** *J Cardiovasc Pharmacol,* 2000, vol. 35, 484-90
- **FOX, KM et al.** *Lancet,* 06 September 2003, vol. 362 (9386), 782-8
- **RIGAT B ; HUBERT C ; ALHENC-GELAS F ; CAMBIEN F ; CORVOL P ; SOUBRIER F.** An insertion/deletion polymorphism in the angiotensin I-converting enzyme gene accounting for half the variance of serum enzyme levels. *J Clin Invest,* 1990, vol. 86, 1343-6
- **CAMBIEN F ; POIRIER O ; LECERF L et al.** Deletion polymorphism in the gene for angiotensin-converting enzyme is a potent risk factor for myocardial infarction. *Nature,* 1992, vol. 359, 641-4
- **COHN JN ; JOHNSON G ; ZIESCHE S et al.** A comparison of enalapril with hydralazine-isosorbide dinitrate in the treatment of chronic congestive heart failure. *N Engl J Med,* 1991, vol. 325, 303-10

- **CUSPIDI C ; MUIESAN ML ; VALAGUSSA L et al.** Comparative effects of candesartan and enalapril on left ventricular hypertrophy in patients with essential hypertension: the candesartan assessment in the treatment of cardiac hypertrophy (CATCH) study. *J Hypertens,* 2002, vol. 20, 2293-300
- **DANSER AH ; DEINUM J ; OSTEROP AP ; ADMIRAAL PJ ; SCHALEKAMP MA.** Angiotensin I to angiotensin II conversion in the human forearm and leg. Effect of the angiotensin converting enzyme gene insertion/deletion polymorphism. *J Hypertens,* 1999, vol. 17, 1867-72
- **EYRIES M ; MICHAUD A ; DEINUM J et al.** Increased shedding of angiotensin-converting enzyme by a mutation identified in the stalk region. *J Biol Chem,* 2001, vol. 276, 5525-32
- **FOX KM.** Efficacy of perindopril in reduction of cardiovascular events among patients with stable coronary artery disease: randomised, double-blind, placebo-controlled, multicentre trial (the EUROPA study). *Lancet,* 2003, vol. 362, 782-8
- **FYHRQUIST F. ; TIKKANEN I. ; GRONHAGEN-RISKA C. ; HORTLING L. ; HICHENS M.** Inhibitor Binding Assay for Angiotensin-Converting Enzyme. *Clin Chemistry,* 1984, vol. 30 (5), 696-700
- **GREENBERG B ; QUINONES MA ; KOILPILLAI C et al.** Effects of long-term enalapril therapy on cardiac structure and function in patients with left ventricular dysfunction. Results of the SOLVD echocardiography substudy. *Circulation,* 1995, vol. 91, 2573-81
- **GRONHAGEN-RISKA C. ; TIKKANEN I. ; FYHRQUIST F.** Competitive inhibitor binding assay (CIBA) of ACE inhibitors. *Acta. Med. Scand. Suppl.,* 1986, vol. 714, 43-47
- **HOOGWERF BJ ; YOUNG JB.** The HOPE study. Ramipril lowered cardiovascular risk, but vitamin E did not. *Cleve Clin J Med,* 2000, vol. 67, 287-93
- **HUBERT C ; HOUOT AM ; CORVOL P ; SOUBRIER F.** Structure of the angiotensin I-converting enzyme gene. Two alternate promoters correspond to evolutionary steps of a duplicated gene. *J Biol Chem,* 1991, vol. 266, 15377-83
- Collaborative Group. *Lancet,* 1995, vol. 345, 669-85
- **KRAMERS C ; DANILOV SM ; DEINUM J et al.** Point mutation in the stalk of angiotensin-converting enzyme causes a dramatic increase in serum angiotensin-converting enzyme but no cardiovascular disease. *Circulation,* 2001, vol. 104, 1236-40
- **LIEBERMAN J ; BEUTLER E.** Elevation of serum angiotensin-converting enzyme in Gaucher's disease. *N Engl J Med,* 1976, vol. 294, 1442-4
- **LIEBERMAN J ; SASTRE A.** An angiotensin-converting enzyme (ACE) inhibitor in human serum. Increased sensitivity of the serum ACE assay for detecting active sarcoidosis. *Chest,* 1986, vol. 90, 869-75
- **LINDPAINTNER K ; PFEFFER MA ; KREUTZ R et al.** A prospective evaluation of an angiotensin-converting-enzyme gene polymorphism and the risk of ischemic heart disease. *N Engl J Med,* 1995, vol. 332, 706-11
- **LINNEBANK M ; KESPER K ; JEUB M et al.** Hereditary elevation of angiotensin converting enzyme suggesting neurosarcoidosis. *Neurology,* 2003, vol. 61, 1819-20
- **LUDERS S ; SCHRADER J ; BERGER J et al.** The PHARAO study: prevention of hypertension with the angiotensin-converting enzyme inhibitor ramipril in patients with high-normal blood pressure: a prospective, randomized, controlled prevention trial of the German Hypertension League. *J Hypertens,* 2008, vol. 26, 1487-96
- **NESTEROVITCH AB ; HOGARTH KD ; ADARICHEV VA et al.** Angiotensin I-converting enzyme mutation (Trp1197Stop) causes a dramatic increase in blood ACE. *PLoS One,* 2009, vol. 4, e8282
- **OPPONG SY ; HOOPER NM.** Characterization of a secretase activity which releases angiotensin-converting enzyme from the membrane. *Biochem J,* 1993, vol. 292, 597-603
- **PFEFFER MA ; BRAUNWALD E ; MOYE LA et al.** Effect of captopril on mortality and morbidity in patients with left ventricular dysfunction after myocardial infarction. Results of the survival and ventricular enlargement trial. The SAVE Investigators. *N Engl J Med,* 1992, vol. 327, 669-77
- **RUGGENENTI P ; FASSI A ; ILIEVA AP et al.** Preventing microalbuminuria in type 2 diabetes. *N Engl J Med,* 2004, vol. 351, 1941-51
- **SEMMLER A ; STEIN RW ; CAPLAN L ; DANILOV SM ; KLOCKGETHER T ; LINNEBANK M.** Hereditary hyper-ACE-emia due to the Pro1199Leu mutation of somatic ACE as a potential pitfall in diagnosis: a first family outside Europe. *Clin Chem Lab Med,* 2006, vol. 44, 1088-9
- Six-month effects of early treatment with lisinopril and transdermal glyceryl trinitrate singly and together withdrawn six weeks after acute myocardial infarction: the GISSI-3 trial. Gruppo Italiano per lo Studio della Sopravvivenza nell'Infarto Miocardico. *J Am Coll Cardiol,* 1996, vol. 27, 337-44
- **STAESSEN JA ; WANG JG ; GINOCCHIO G et al.** The deletion/insertion polymorphism of the angiotensin converting enzyme gene and cardiovascular-renal risk. *J Hypertens,* 1997, vol. 15, 1579-92

- **STUDDY P ; BIRD R ; JAMES DG.** Serum angiotensin-converting enzyme (SACE) in sarcoidosis and other granulomatous disorders. *Lancet,* 1978, vol. 2, 1331-4
- **VAN DIJK MA ; KROON I ; KAMPER AM ; BOOMSMA F ; DANSER AH ; CHANG PC.** The angiotensin-converting enzyme gene polymorphism and responses to angiotensins and bradykinin in the human forearm. *J Cardiovasc Pharmacol,* 2000, vol. 35, 484-90
- **WEI L ; ALHENC-GELAS F ; SOUBRIER F ; MICHAUD A ; CORVOL P ; CLAUSER E.** Expression and characterization of recombinant human angiotensin I-converting enzyme. Evidence for a C-terminal transmembrane anchor and for a proteolytic processing of the secreted recombinant and plasma enzymes. *J Biol Chem,* 1991, vol. 266, 5540-6